# EUROPEAN PATENT APPLICATION

(11) **EP 2 319 841 A1**
(43) Date of publication of application: **11.05.2011**
(21) Application number: 09802838.4
(22) Date of filing: 14.07.2009
(51) Int. Cl.: C07D 401/06, A61K 31/4525, A61K 31/454, A61K 31/4545, A61P 1/00, A61P 1/16, A61P 3/04, A61P 3/06, A61P 3/10, A61P 3/12, A61P 5/00, A61P 9/00, A61P 9/10, A61P 11/00

(54) **(5-MEMBERED)-(5-MEMBERED) OR (5-MEMBERED)-(6-MEMBERED) FUSED RING CYCLOALKYLAMINE DERIVATIVE**

(30) Priority: 30.07.2008 JP 2008195814
(71) Applicant: MSD K.K., Chiyoda-ku Tokyo 102-8667 (JP)
(72) Inventor: KISHINO Hiroyuki, Tokyo 102-8667 (JP); SATO Nagaaki, Tokyo 102-8667 (JP); SUZUKI Takao, Tokyo 102-8667 (JP)
(74) Representative: Buchan, Gavin MacNicol
(86) International application number: PCT/JP2009/062727
(87) International publication number: WO 2010/013595

(57) **Abstract**

Disclosed are a compound of a formula (I) and its pharmaceutically-acceptable salt: 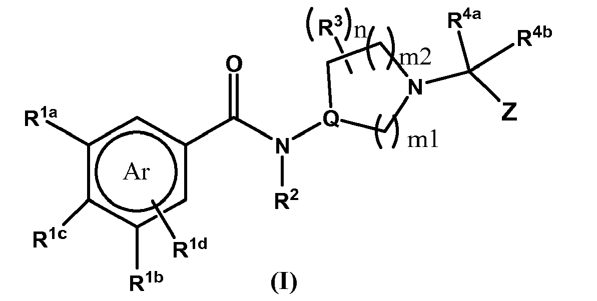 wherein Q is CH or N; R^{1a}, R^{1b}, R^{1c} and R^{1d} are independently a C₁₋₆ alkyl, a halo-C₁₋₆ alkyl, etc.; R² is a hydrogen atom, etc.; R³ is independently a hydrogen atom, a C₁₋₆ alkyl, etc., or two R³'S, taken together, form a bridge such as methylene, etc.; R⁴ and R^{4b} are independently a hydrogen atom, a C₁₋₆ alkyl, etc.; Z is a bicyclic aromatic hetero ring, etc.; Ar is a benzene ring, etc.; m1 and m2 are independently 0, 1 or 2; n is an integer of from 1 to 4. The compound and its salt act as a melanin concentrating hormone receptor antagonist, and are useful as a preventive or remedy for central system disorders, cardiovascular disorders, metabolic disorders, etc.

## Description

### TECHNICAL FIELD

The present invention relates to a novel 5/5- or 5/6-membered condensed ring cycloalkylamine derivative. The compound acts as a melanin concentrating hormone receptor antagonist, and is useful as a preventive or remedy for various circular system diseases, nervous system diseases, metabolic diseases, genital diseases, respiratory diseases, digestive diseases, etc.

### BACKGROUND ART

Melanin concentrating hormone (hereafter referred to as "MCH") is a cyclic peptide hormone/neuro-peptide, which was for the first time isolated by Kawauchi, et al., in 1983 from sermon hypophysis [see Nature, Vol. 305, 321 (1983)]. The hormone is known to functionally antagonize for melanin cell stimulating hormone in fishes, to cause concentration of melanin granules in melanophore and participate in body color change [see International Review of Cytology, Vol. 126, 1 (1991); Trends in Endocrinology and Metabolism, Vol. 5, 120 (1994)]. Also in mammals, MCH-containing neuron cells are localized in the hypothalamus lateral field and uncertain zone, but their nerve fibers project over a very wide scope in the brain [see The Journal of Comparative Neurology, Vol. 319, 218 (1992)], and MCH is considered to preside over various central functions in living bodies.

Hypothalamus lateral field is known of old as a feeding center, and furthermore, recently, molecular biological and pharmacological knowledge suggesting participation of MCH in controlling energetic homeostasis are much accumulated. That is, it is reported that expression of mRNA, which is an MCH precursor, is accelerated in the brains of ob/ob mice, db/db mice, KKAy mice, Zucker fatty rats which are model animals of hereditary obesity, and in the brains of fasting mice [see Nature, Vol. 380, 243 (1996); Diabetes, Vol. 47, 294 (1998); Biochemical and Biophysical Research Communications, Vol. 268, 88 (2000); Molecular Brain Research, Vol. 92, 43 (2001)].

Acute ventricular administration of MCH to rats was observed to induce accelerated feeding activity [Nature, Vol. 380, 243 (1996)] and chronic administration thereof invites obesity accompanied by polyphagy [see Proceedings of the National Academy of Sciences of the United States of America, Vol. 99, 3240 (2002)]. Moreover, MCH precursor gene-deficient mice show reduced feed ingestion or rise in oxygen consumption per body weight compared to wild type mice, and their low body weight due to decrease in body fat was observed [see Nature, Vol. 396, 670 (1998)].

On the other hand, transgenic mice which express excessive MCH precursor develop obesity accompanied by polyphagy and insulin resistance [see The Journal of Clinical Investigation, Vol. 107, 379 (2001)]. Consequently, it is suggested that MCH is an important factor for developing obesity and participates in diseases induced by metabolic disorders or respiratory diseases for which obesity is one risk factor. Besides, MCH is known to participate also in anxiety-causing action, epilepsy, memory, learning, diuretic action, sodium/potassium excretory action, oxytocin secreting action, reproduction and reproductive function [see Peptides, Vol. 17, 171 (1996); Peptides, Vol. 18, 1095 (1997); Peptides, Vol. 15, 757 (1994); Journal of Neuroendocrinology, Vol. 8, 57 (1996); Critical Reviews in Neurobiology, Vol. 8, 221 (1994)].

MCH causes versatile pharmacological actions through MCH receptors which are present mainly in the central nervous system. As receptors of MCH, at least two types of type 1 receptors (MCH-1R or SLC-1) and type 2 receptors (MCH-2R or SLT) are known [see Nature, Vol. 400, 261 (1999); Nature, Vol. 400, 265 (1999); Biochemical and Biophysical Research Communications, Vol. 261, 622 (1999); Nature Cell Biology, Vol. 1, 267 (1999); FEBS Letters, Vol. 457, 522 (1999); Biochemical and Biophysical Research Communications, Vol. 283, 1013 (2001); The Journal of Biological Chemistry, Vol. 276, 20125 (2001); Proceedings of the National Academy of Sciences of the United States of America, Vol. 98, 7564 (2001); Proceedings of the National Academy of Sciences of the United States of America, Vol. 98, 7576 (2001); The Journal of Biological Chemistry, Vol. 276, 34664 (2001); Molecular Pharmacology, Vol. 60, 632 (2001)].

Of those, the pharmacological action observed on rodents is induced mainly via MCH-1R [see Genomics, Vol. 79, 785 (2002)]. Because MCH-1R gene-deficient mice chronically administered with MCH do not develop polyphagy or obesity, it is known that controlling of energy metabolism by MCH is induced via MCH-1R. Furthermore, the deficiency of MCH-1R is known to promote the activity amount of mice [see Proceedings of the National Academy of Sciences of the United States of America, Vol. 99, 3240 (2002)], and its participation in central system diseases accompanied by behavioral disorders, for example, attention-deficit hyperactivity disorder, schizophrenia, depression and the like also is strongly suggested [see Molecular Medicine Today, Vol. 6, 43 (2000); Trends in Neuroscience, Vol. 24, 527 (2001)].

It is also reported that an autoantibody to MCH-1R is present in serum of vitiligo vulgaris patients [see The Journal of Clinical Investigation, Vol. 109, 923 (2002)]. Furthermore, expression of MCH-1R in certain species of cancer cells was reported, and in vivo MCH and MCH-1R expression sites also suggest MCH's participation in cancer, sleep, vigil, drug dependence and digestive disorders [see Biochemical and Biophysical Research Communications, Vol. 289, 44 (2001); Neuroendocrinology, Vol. 61, 348 (1995); Endocrinology, Vol. 137, 561 (1996); The Journal of Comparative Neurology, Vol. 435, 26 (2001)].

Functions of MCH are expressed upon its binding to MCH receptors. Therefore, when its binding to MCH receptor is inhibited, then expression of MCH action can be inhibited. In consequence, substances which are antagonists for binding of MCH with its receptor are expected to be useful as preventive or remedy for those various diseases in which MCH participates, for example, metabolic disorders such as obesity, diabetes, hormone disorder, hyperlipidemia, gout, fatty liver, etc.; cardiovascular disorders such as stenocardia, acute/congestive heart failure, myocardial infarction, coronary atherosclerosis, renal diseases, electrolyte abnormality, etc.; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism, etc.; reproductive disorders such as infertility, preterm labor, sexual dysfunction, etc.; and other digestive disorders, respiratory disorders, cancer or pigmentation.

As compounds having an MCH receptor-antagonistic effect, for example, Non-Patent Reference 1 discloses compounds having 3, 4-methylenedioxybenzyl bonding to the 1-position of a piperidine skeleton; and Patent Reference 1 and Patent Reference 2 disclose derivatives having naphthylmethyl bonding to the 1-position of a piperidine skeleton.
Non-Patent Reference 1: Bioorganic Medicinal Chemistry Letters, Vol. 15, pp. 4174-4179, 2005
Patent Reference 1: WO2008/1
Patent Reference 2: WO2008/44632

### DISCLOSURE OF THE INVENTION

### TECHNICAL PROBLEMS

The present inventors have assiduously studied compounds having an MCH receptor-antagonistic effect and, as a result, have found that a 5/5-membered or 5/6-membered condensed ring cycloalkylamine derivative having a 5/5-membered or 5/6-membered bicyclic heteroaryl ring bonding to the 1-position of a piperidine ring via methylene and having a specific substituent at the 4-position thereof not only has a remarkable MCH1R-antagonistic effect but also has high selectivity to bonding to other receptors, and further can be a compound excellent in the metabolic stability thereof; and on the basis of these findings, the inventors have completed the present invention.

Specifically, the invention provides the following:
(1) A compound of a formula (I) or a pharmaceutically-acceptable salt thereof: 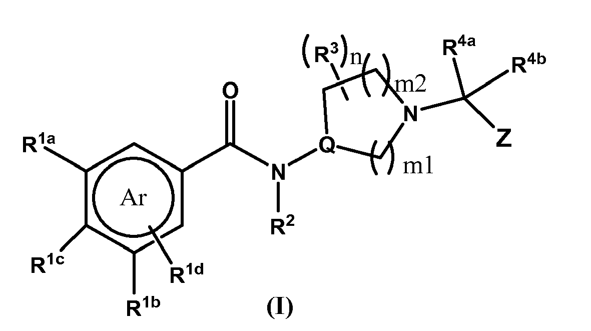 [wherein,
   Q represents CH or N;
   R^{1a} and R^{1b} each independently represent a halogen, a C₁₋₆ alkyloxy or a halo-C₁-₆ alkyloxy;
   R¹ represents a halogen, a C₁₋₆ alkyl or a halo-C₁₋₆ alkyl;
   R^{1d} represents a hydrogen atom, a halogen, a C₁₋₆ alkyl, a halo-C₁₋₆ alkyl, a C₁₋₆ alkyloxy, a halo-C₁₋₆ alkyloxy, a cyano, a hydroxy-C₁₋₆ alkyl, a C₃₋₈ cycloalkyl or a hydroxy-C₃₋₈ cyclo alkyl;
   R² represents a hydrogen atom, a C₁₋₆ alkyl or a halo-C₁₋₆ alkyl;
   R³ each independently represents a hydrogen atom, a halogen, a C₁₋₆ alkyl or a halo-C₁₋₆ alkyl; or in case where two R³'s are on different carbon atoms, they form, taken together, a substituent selected from a group of methylene, ethylene, propylene and -CH₂-O-CH₂-, or in case where two R³'s are on the same carbon atom, they form, taken together, an oxo;
   R^{4a} and R^{4b} each independently represent a hydrogen atom, a C₁₋₆ alkyl or a halo-C₁₋₆ alkyl;
   Z represents 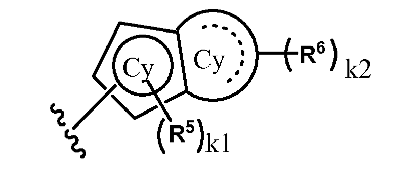 (wherein R⁵ each independently represents a hydrogen atom, a C₁₋₆ alkyl, a halo-C₁₋₆ alkyl, a C₂₋₆ alkenyl, a C₂₋₆ alkynyl, a cyano, a hydroxy-C₁₋₆ alkyl, a C₃₋₈ cycloalkyl, a hydroxy-C₃₋₈ cycloalkyl, a C₁₋₆ alkyloxycarbonyl, an aryl, a heteroaryl, an aralkyl, a heteroaralkyl or an arylsulfonyl; wherein the aryl, or the heteroaryl, or the aryl or the heteroaryl in the aralkyl, the heteroaralkyl or the arylsulfonyl may be substituted with a halogen;
   R⁶ each independently represents a hydrogen atom, a halogen, a C₁₋₆ alkyl, a C₃₋₈ cycloalkyl, a halo-C₁₋₆ alkyl, a C₁₋₆ alkyloxy, a halo-C₁₋₆ alkyloxy, a C₁₋₆ alkyloxycarbonyl, a C₁₋₆ alkyloxy-C₁₋₆ alkyl, an aralkyl, an aralkyloxy, a heteroaryl, an arylthio or an arylsulfonyl; or in case where two R⁶'s are on the same hydrogen atom, they form, taken together, an oxo; k1 indicates 1 or 2; k2 indicates 1, 2, 3 or 4; 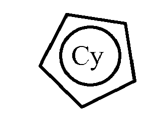 represents a 5-membered unsaturated carbon ring, or a 5-membered heteroaryl ring containing from 1 to 3, the same or different hetero atoms selected from a nitrogen atom, an oxygen atom and a sulfur atom; 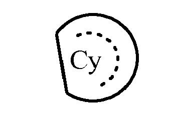 represents a 6-membered unsaturated nitrogen-containing hetero ring, a 5- or 6-membered aryl ring, or a 6-membered heteroaryl ring); 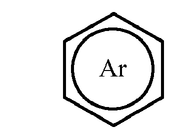 represents a benzene ring, a pyridine ring or a pyrimidine ring;
   m1 and m2 each independently indicate 0, 1 or 2
   n indicates an integer of from 1 to 4].
   Further, the invention provides the following:
(2) A melanin concentrating hormone receptor antagonist comprising a compound of (1) or a pharmaceutically-acceptable salt thereof as the active ingredient;
(3) A pharmaceutical composition containing a medicinally-acceptable additive and a compound of (1) or a pharmaceutically-acceptable salt thereof;
(4) A preventive or remedy comprising a compound of (1) or a pharmaceutically-acceptable salt thereof as the active ingredient, for obesity, diabetes, fatty liver, bulimia, depression or anxiety;
(5) A pharmaceutical composition based on an MCH1R receptor antagonistic effect, comprising a compound of (1) or a pharmaceutically-acceptable salt thereof as the active ingredient.

The invention is described in more detail hereinunder.

"Halogen" includes fluorine, chlorine, bromine and iodine.

"C₁₋₆ alkyl" includes a linear alkyl having from 1 to 6 carbon atoms or a branched alkyl having from 3 to 6 carbon atoms, concretely, for example, methyl, ethyl, n-propyl, isopropyl, n-butyl, isobutyl, sec-butyl, tert-butyl, n-pentyl, isopentyl, neopentyl, tert-amyl, 2-propyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl, 1-ethyl-1-methylpropyl.

"C₃₋₈ cycloalkyl " means a cycloalkyl having from 3 to 8 carbon atoms, including cyclopropyl, cyclobutyl, cyclopentyl, cyclohexyl, cycloheptyl, cyclooctyl.

"Halo-C₁₋₆ alkyl " includes a C₁₋₆ alkyl in which a part or all of the hydrogen atoms are substituted with halogen, for example, including fluoromethyl, difluoromethyl, trifluoromethyl, 2-fluoroethyl, 1,2-difluoroethyl.

"C₂₋₆ alkenyl" includes a linear or branched alkenyl having from 2 to 6 carbon atoms, for example, vinyl, 1-propenyl, 2-propenyl, isopropenyl, 3-butenyl, 2-butenyl, 1-butenyl.

"C₂₋₆ alkynyl" includes a linear or branched alkynyl having from 2 to 6 carbon atoms, for example, 1-propynyl, 2-propynyl.

"C₁₋₆ alkyloxy" includes a group of a C₁₋₆ alkyl bonding to an oxygen atom, concretely methoxy, ethoxy, n-propyloxy, isopropyloxy, n-butoxy, isobutoxy, tert-butoxy, n-pentyloxy.

"Halo-C₁₋₆ alkyloxy" includes a group of a halo-C₁₋₆ alkyl bonding to an oxygen atom, concretely fluoromethoxy, chloromethoxy, difluoromethoxy, dichloromethoxy, trifluoromethoxy, trichloromethoxy, 2-fluoroethoxy, 1,2-difluoroethoxy.

"Hydroxy-C₁₋₆ alkyl " is a C₁₋₆ alkyl in which a part of the hydrogen atoms are substituted with hydroxy, concretely including, for example, hydroxymethyl, 2-hydroxyethyl.

"Hydroxy-C₃₋₆ cycloalkyl " is a C₃₋₆ cycloalkyl in which a part of the hydrogen atoms are substituted with hydroxy, concretely including, for example, hydroxycyclopropyl, hydroxycyclobutyl.

"C₁₋₆ alkyloxy-C₁₋₆ alkyl" is a C₁₋₆ alkyl substituted with a C₁₋₆ alkyloxy, concretely including, for example, methoxymethyl, ethoxymethyl, n-propyloxymethyl, isopropyloxymethyl, 1-methoxyethyl, 2-methoxyethyl.

"C₁₋₆ alkyloxycarbonyl" is a C₁₋₆ alkyloxy bonding to a carbonyl (-CO-) and includes an alkyloxycarbonyl having from 1 to 6 carbon atoms, concretely, for example, methoxycarbonyl, ethoxycarbonyl, n-propyloxycarbonyl, isopropyloxycarbonyl, n-butoxycarbonyl, isobutoxycarbonyl, tert-butoxycarbonyl, n-pentyloxycarbonyl.

"C₁₋₆ alkylcarbonyl" is a group of a C₁₋₆ alkyl bonding to a carbonyl, and includes an alkylcarbonyl having from 1 to 6 carbon atoms, concretely, for example, acetyl, propionyl, butyryl, isobutyryl, valeryl, isovaleryl, pivaloyl.

"Aryl" includes, for example, phenyl, naphthyl.

"Heteroaryl" means a 5-membered or 6-membered monocyclic heteroaryl having the same or different, one or more, preferably from 1 to 3 hetero atoms selected from a group consisting of an oxygen atom, a nitrogen atom and a sulfur atom, or a condensed cyclic heteroaryl formed through condensation of the monocyclic heteroaryl and the above-mentioned aryl, or through condensation of the same or different monocyclic heteroaryls; and this includes, for example, pyrrolyl, furyl, thienyl, imidazolyl, pyrazolyl, thiazolyl, isothiazolyl, oxazolyl, isoxazolyl, triazolyl, tetrazolyl, oxadiazolyl, 1,2,3-thiadiazolyl, 1,2,4-thiadiazolyl, 1,3,4-thiadiazolyl, pyridyl, pyrazinyl, pyrimidinyl, pyridazinyl, 1,2,4-triazinyl, 1,3,5-triazinyl, indolyl, benzofuranyl, benzothienyl, benzimidazolyl, benzopyrazolyl, benzoxazolyl, benzisoxazolyl, benzothiazolyl, benzisothiazolyl, indazolyl, purinyl, quinolyl, isoquinolyl, phthalazinyl, naphthyridinyl, quinoxalinyl, quinazolinyl, cinnolinyl, puteridinyl, pyrido[3,2-b]pyridyl.

Of the above-mentioned heteroaryl, the 5-membered sulfur-containing heteroaryl ring optionally having a nitrogen atom in the ring includes thienyl, thiazolyl, isothiazolyl, 1,2,3-thiadiazolyl, 1,2,4-thiadiazolyl, 1,3,4-thiadiazolyl;
the 5-membered nitrogen-containing heteroaryl ring includes pyrrolyl, imidazolyl, pyrazolyl, thiazolyl, isothiazolyl, triazolyl, tetrazolyl, oxadiazolyl, 1,2,3-thiadiazolyl, 1,2,4-thiadiazolyl, 1,3,4-thiadiazolyl;
the 5-membered heteroaryl ring having the same or different, from 1 to 3 hetero atoms elected from a nitrogen atom, an oxygen atom and a sulfur atom includes pyrrolyl, furyl, thienyl, imidazolyl, pyrazolyl, thiazolyl, isothiazolyl, oxazolyl, isoxazolyl, triazolyl, tetrazolyl, oxadiazolyl, 1,2,3-thiadiazolyl, 1,2,4-thiadiazolyl, 1,3,4-thiadiazolyl;
the 6-membered nitrogen-containing heteroaryl ring includes pyridyl, pyrazinyl, pyrimidinyl, pyridazinyl, 1,2,4-triazinyl, 1,3,5-triazinyl.

The 6-membered unsaturated nitrogen-containing hetero ring includes dihydropyridine.

"Aryloxy" is a group of the above-mentioned aryl bonding to an oxygen atom.

"Heteroaryloxy" is a group of the above-mentioned heteroaryl bonding to an oxygen atom.

"Arylsulfonyl" is a group of the above-mentioned aryl bonding to a sulfonyl.

"Heteroarylsulfonyl" is a group of the above-mentioned heteroaryl bonding to a sulfonyl.

"Aralkyl" is a group of the above-mentioned aryl bonding to the above-mentioned C₁₋₆ alkyl, including benzyl, 1-phenylethyl, 2-phenylethyl, 1-naphthylmethyl, 2-naphthylmethyl.

"Heteroaralkyl" is a group of the above-mentioned heteroaryl bonding to the above-mentioned C₁₋₆ alkyl.

"Aralkyloxy" is a group of the above-mentioned aralkyl bonding to an oxygen atom.

"Pharmaceutically-acceptable salts" of a derivative of formula (I) mean ordinary salts those are acceptable as medicines. Their examples are acid-addition salts to the amine moiety of the compound of formula (I) or acid-addition salts to the nitrogen-containing hetero ring thereof, or base-addition salts to the acidic substituent, if any, of the compound of formula (I).

The acid-addition salts include inorganic acid salts such as hydrochlorides, sulfates, nitrates, phosphates, perchlorates; organic acid salts such as maleates, fumarates, tartrates, citrates, ascorbates, trifluoroacetates; and sulfonates such as methanesulfonates, isothiocyanates, benzenesulfonates, p-toluenesulfonates.

The base-addition salts include alkali metal salts such as sodium salts, potassium salts; alkaline earth metal salts such as calcium salts, magnesium salts; ammonium salts; and organic amine salts such as trimethylamine salts, triethylamine salts, dicyclohexylamine salts, ethanolamine salts, diethanolamine salts, triethanolamine salts, procaine salts, N,N'-dibenzylethylenediamine salts.

For the purpose of more concretely disclosing the derivatives of the invention hereinunder, various symbols used in formula (I) are described in detail with reference to their examples.

Ar represents a benzene ring, a pyridine ring or a pyrimidine ring.

Ar is preferably a benzene ring.

R^{1a} and R^{1b} each independently represent a halogen, a C₁₋₆ alkyloxy or a halo-C₁₋₆ alkyloxy;

R¹ represents a halogen, a C₁₋₆ alkyl or a halo-C₁₋₆ alkyl;

R^{1d} represents a hydrogen atom, a halogen, a C₁₋₆ alkyl, a halo-C₁₋₆ alkyl, a C₁₋₆ alkyloxy, a halo-C₁₋₆ alkyloxy, a cyano, a hydroxy-C₁₋₆ alkyl, a C₃₋₈ cycloalkyl or a hydroxy-C₃₋₈ cycloalkyl.

Concretely, R^{1a} and R^{1b} are independently a halogen such as fluorine, chlorine; C₁₋₆ alkyloxy such as methoxy, ethoxy, n-propyloxy, isopropyloxy; or a halo-C₁₋₆ alkyloxy such as chloromethoxy, trichloromethoxy, fluoromethoxy, trifluoromethoxy, fluoroethoxy, fluoropropyloxy.

Concretely, R^{1c} is a halogen such as fluorine, chlorine; a C₁₋₆ alkyl such as methyl, ethyl, n-propyl, isopropyl, n-butyl; or a halo-C₁₋₆ alkyl such as chloromethyl, trichloromethyl, fluoromethyl, trifluoromethyl, chloroethyl, fluoroethyl.

Concretely, R^{1d} is a hydrogen atom; a halogen such as fluorine, chlorine; a C₁₋₆ alkyl such as methyl, ethyl; a halo-C₁₋₆ alkyl such as chloromethyl, fluoromethyl, trifluoromethyl; a C₁₋₆ alkyloxy such as methoxy, ethoxy; a halo-C₁₋₆ alkyloxy such as chloromethoxy, fluoromethoxy, trifluoromethoxy; a cyano; a hydroxy-C₁₋₆ alkyl such as hydroxymethyl, hydroxyethyl; a C₃₋₈ cycloalkyl such as cyclopropyl, cyclobutyl, cyclopentyl, cyclohexyl; a hydroxy-C₃₋₈ cycloalkyl such as hydroxycyclopropyl, hydroxycyclobutyl.

Preferably, R¹ and R^{1b} each are a halogen or a C₁₋₆ alkyloxy, more preferably methoxy or ethoxy.

Preferably, R^{1c} is a halogen, a C₁₋₆ alkyl or a halo-C₁₋₆ alkyl, more preferably a halogen or a C₁₋₆ alkyl, even more preferably a C₁₋₆ alkyl (especially methyl).

As the combination of R^{1a}, R^{1b} and R^{1c}, preferred is as follows: R^{1a} and R^{1b} are independently a C₁₋₆ alkyloxy, and R^{1c} is a C₁₋₆ alkyl.

Preferably, R^{1d} is a substituent selected from a hydrogen atom, a halogen, a C₁₋₆ alkyl, a halo-C₁₋₆ alkyl and a C₁₋₆ alkyloxy, more preferably a hydrogen atom or a halogen.

R² is a hydrogen atom, a C₁₋₆ alkyl or a halo-C₁₋₆ alkyl.

Concretely, R² is a hydrogen atom; a halogen such as fluorine, chlorine; a C₁₋₆ alkyl such as methyl, ethyl; or a halo-C₁₋₆ alkyl such as chloromethyl, fluoromethyl, trifluoromethyl; and is preferably a hydrogen atom.

R³ is independently a hydrogen atom, a halogen, a C₁₋₆ alkyl or a halo-C₁₋₆ alkyl; or in case where two R³'s are on different carbon atoms, they form, taken together, a substituent selected from a group of methylene, ethylene, propylene and -CH₂ -O-CH₂ -, or in case where two R³'s are on the same carbon atom, they form, taken together, an oxo.

Concretely, R³ is a hydrogen atom; a C₁₋₆ alkyl such as methyl, ethyl; or a halo-C₁_ ₆ alkyl such as chloromethyl, fluoromethyl; or represents an oxo; or in case where two R³'s are on different carbon atoms, they form, taken together, a bridge such as methylene, ethylene, propylene or -CH₂ -O-CH₂-.

Preferably, R³ is a hydrogen atom.

n is an integer of from 1 to 4. n is preferably 1 or 2, more preferably 1.

R⁴ and R^{4b} are independently a hydrogen atom, a C₁₋₆ alkyl or a halo-C₁₋₆ alkyl.

Concretely, R⁴ and R^{4b} are a hydrogen atom; a C₁₋₆ alkyl such as methyl, ethyl, propyl; or a halo-C₁₋₆ alkyl such as chloromethyl, fluoromethyl; and are preferably both hydrogen atoms.

m1 and m2 are independently 0, 1 or 2.

m1 and m2 are preferably both 2.

Q is CH or N.

Q is preferably CH.

Accordingly, the formula, 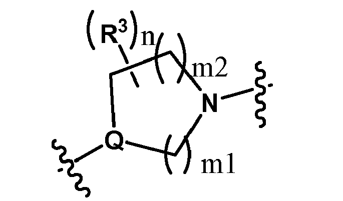 [wherein the symbols are the same as above] includes the following: 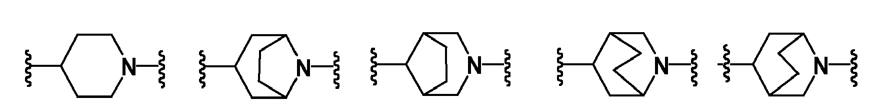 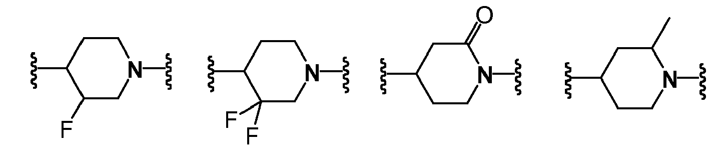 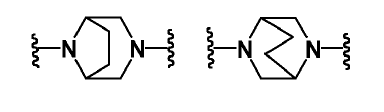 Z represents 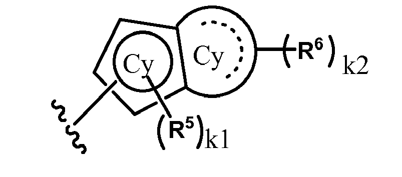 (wherein R⁵ each independently represents a hydrogen atom, a C₁₋₆ alkyl, a halo-C₁₋₆ alkyl, a C₂₋₆ alkenyl, a C₂₋₆ alkynyl, a cyano, a hydroxy-C₁₋₆ alkyl, a C₃₋₈ cycloalkyl, a hydroxy-C₃₋₈ cycloalkyl, a C₁₋₆ alkyloxycarbonyl, an aryl, a heteroaryl, an aralkyl, a heteroaralkyl or an arylsulfonyl; wherein the aryl, or the heteroaryl, or the aryl or the heteroaryl in the aralkyl, the heteroaralkyl or the arylsulfonyl may be substituted with a halogen;

R⁶ each independently represents a hydrogen atom, a halogen, a C₁₋₆ alkyl, a C₃₋₈ cycloalkyl, a halo-C₁₋₆ alkyl, a C₁₋₆ alkyloxy, a halo-C₁₋₆ alkyloxy, a C₁₋₆ alkyloxycarbonyl, a C₁₋₆ alkyloxy-C₁₋₆ alkyl, an aralkyl, an aralkyloxy, a heteroaryl, an arylthio or an arylsulfonyl; or in case where two R⁶'s are on the same hydrogen atom, they form, taken together, an oxo;
k1 indicates 1 or 2; k2 indicates 1, 2, 3 or 4; 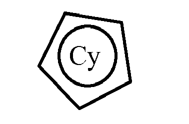 represents a 5-membered unsaturated carbon ring, or a 5-membered heteroaryl ring containing from 1 to 3, the same or different hetero atoms selected from a nitrogen atom, an oxygen atom and a sulfur atom; 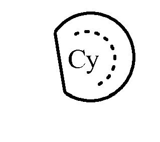 represents a 6-membered unsaturated nitrogen-containing hetero ring, 6-membered aryl ring, or a 5- or 6-membered heteroaryl ring).

Concretely, Z includes the following formulae (Z-1), (Z-2), (Z-3), (Z-4), (Z-5) and (Z-6): 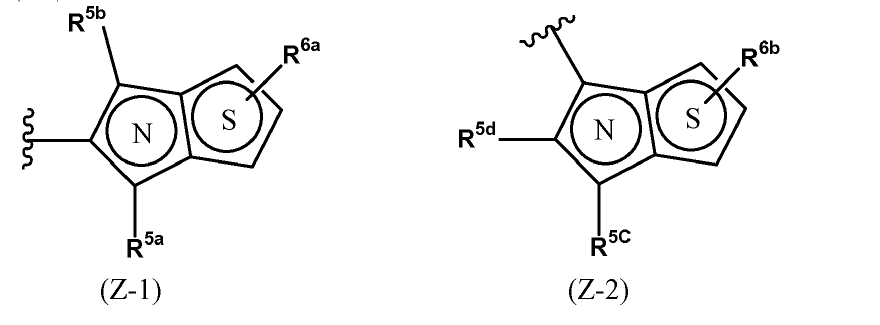 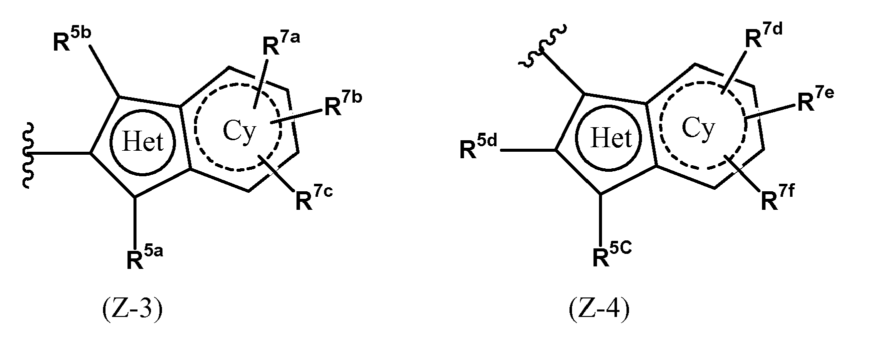 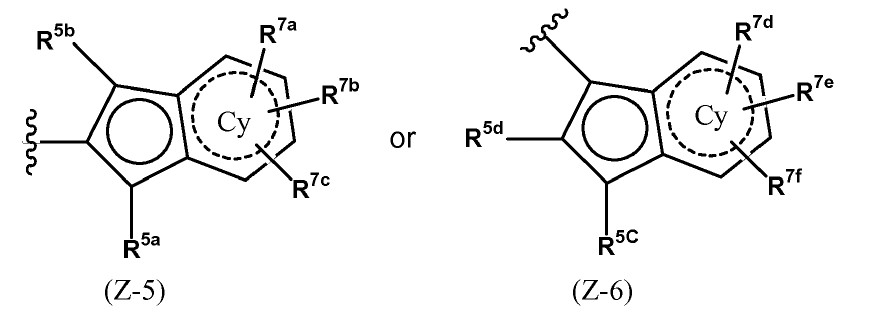
[In the formulae, R^{5a}, R^{5b}, R⁵ and R^{5d} each independently represent a hydrogen atom, a C₁₋₆ alkyl, a halo-C₁₋₆ alkyl, a C₂₋₆ alkenyl, a C₂₋₆ alkynyl, a cyano, a hydroxy-C₁₋₆ alkyl, a C₃₋₈ cycloalkyl, a hydroxy-C₃₋₈ cycloalkyl, a C₁₋₆ alkyloxycarbonyl, an aryl, a heteroaryl, an aralkyl, a heteroaralkyl or an arylsulfonyl, wherein the aryl, the heteroaryl, or the aryl or the heteroaryl in the aralkyl, the heteroaralkyl or the arylsulfonyl may be substituted with a halogen;
R⁶ and R^{6b} each independently represent a hydrogen atom, a halogen, a C₁₋₆ alkyl, a C₃₋₈ cycloalkyl, a halo-C₁₋₆ alkyl, a C₁₋₆ alkyloxy, a halo-C₁₋₆ alkyloxy, a C₁₋₆ alkyloxycarbonyl, a C₁₋₆ alkyloxy-C₁₋₆ alkyl, an aralkyl, an aralkyloxy, a heteroaryl, an arylthio or an arylsulfonyl;
R^{7a} , R^{7b}, R^{7c}, R^{7d}, R^{7e} and R^{7f} each independently represent a hydrogen atom, a halogen, a C₁₋₆ alkyl, a C₃₋₈ cycloalkyl, a halo-C₁₋₆ alkyl, a C₁₋₆ alkyloxy, a halo-C₁₋₆ alkyloxy, a C₁₋₆ alkyloxycarbonyl, a C₁₋₆ alkyloxy-C₁₋₆ alkyl, an aralkyl, an aralkyloxy, a heteroaryl, an arylthio or an arylsulfonyl; or in case where R^{7a} and R^{7b}, or R^{7d} and R^{7e} are on the same carbon atom, they form, taken together, an oxo; 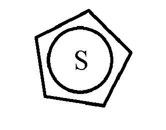 represents a 5-membered sulfur-containing heteroaryl ring optionally having a nitrogen atom in the ring; 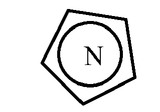 represents a 5-membered nitrogen-containing heteroaryl ring; 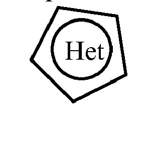 represents a 5-membered heteroaryl ring having the same or different, from 1 to 3 hetero atoms selected from a nitrogen atom, an oxygen atom and a sulfur atom; 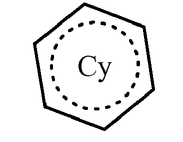 represents a 6-membered unsaturated nitrogen-containing hetero ring, a 6-membered aryl ring or a 6-membered nitrogen-containing heteroaryl ring].

Preferably, R^{5a}, R^{5b} , R⁵ and R^{5d} each are a hydrogen atom; a C₁₋₆ alkyl such as methyl, ethyl, n-propyl, isopropyl, 2-methylpropyl; a halo-C₁₋₆ alkyl such as chloromethyl, fluoromethyl, trifluoromethyl; a C₂₋₆ alkenyl such as 2-propenyl; a C₂₋₆ alkynyl such as 2-propynyl; a cyano; a hydroxy-C₁₋₆ alkyl such as hydroxymethyl; a C₃₋₈ cycloalkyl such as cyclopentyl, cyclohexyl; a hydroxy-C₃₋₈ cycloalkyl such as hydroxycyclopropyl; a C₁₋₆ alkyloxycarbonyl such as methyloxycarbonyl, ethyloxycarbonyl, t-butyloxycarbonyl; an aryl such as phenyl, 4-chlorophenyl, 4-fluorophenyl; a heteroaryl such as pyridyl, 6-chloro-4-trifluoromethylpyridyl; an aralkyl such as benzyl, 4-chlorobenzyl, 4-fluorobenzyl; a heteroaralkyl such as 6-chloro-3-pyridinylmethyl, 2-chloro-1,3-thiazol-5-ylmethyl; an arylsulfonyl such as benzenesulfonyl, toluenesulfonyl.

Preferably, R⁶ and R^{6b} each are a hydrogen atom; a halogen such as fluorine, chlorine; a C₁₋₆ alkyl such as methyl, ethyl, n-propyl, isopropyl; a C₃₋₈ cycloalkyl such as cyclopentyl, cyclohexyl; a halo-C₁₋₆ alkyl such as chloromethyl, fluoromethyl, trifluoromethyl; a C₁₋₆ alkyloxy such as methoxy, ethoxy; a halo-C₁₋₆ alkyloxy such as trifluoromethoxy; a C₁₋₆ alkyloxycarbonyl such as methyloxycarbonyl, ethyloxycarbonyl, t-butyloxycarbonyl; a C₁₋₆ alkyloxy-C₁₋₆ alkyl such as methoxymethyl, methoxyethyl; an aralkyl such as benzyl, 4-chlorobenzyl, 4-fluorobenzyl; an aralkyloxy such as benzyloxy; a heteroaryl such as pyridyl; an arylthio such as phenylthio; an arylsulfonyl such as benzenesulfonyl, toluenesulfonyl; or R^{6a} and R^{6b}, taken together, form an oxo.

Preferred examples of R^{7a}, R^{7b}, R^{7c}, R^{7d}, R^{7e} and R^{7f} are the same as those of R^{6a} and R^{6b}.

Preferably, formulae (Z-1) and (Z-2) include the following: 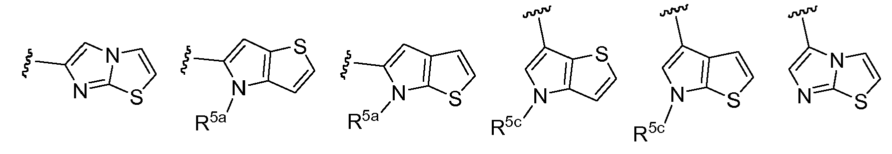 [In the formulae, the symbols are the same as above.]

More preferably, formulae (Z-1) and (Z-2) are the following: 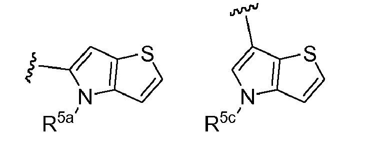 [In the formulae, the symbols are the same as above.]

Preferably, formulae (Z-3) and (Z-4) include the following: 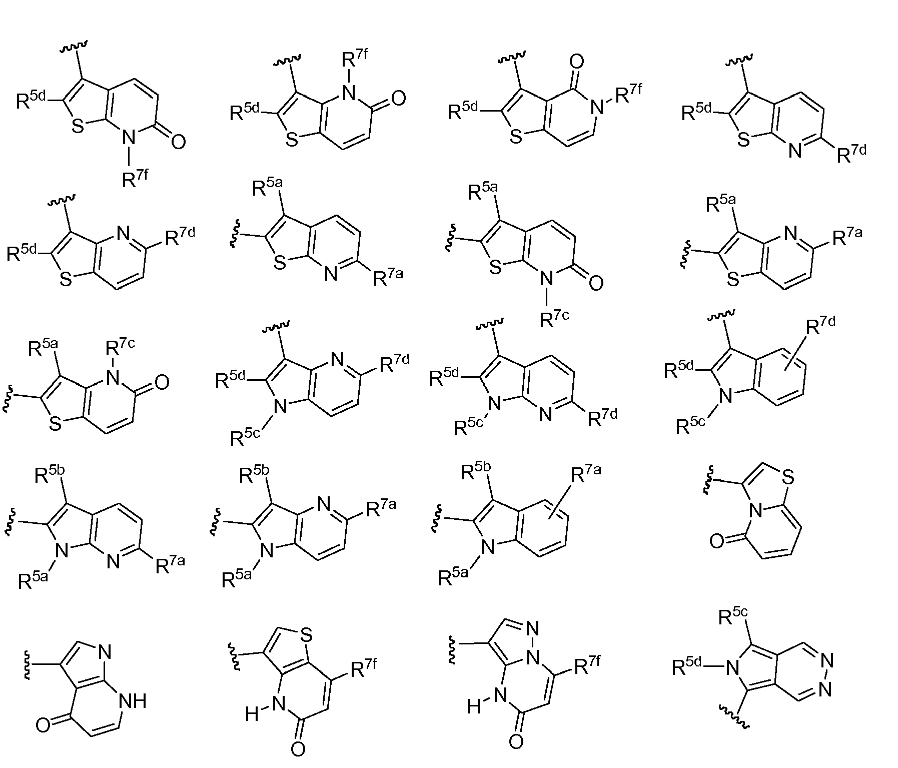 [In the formulae, the symbols are the same as above.]

Of the substituents of formulae (Z-3) and (Z-4), more preferred are the following: 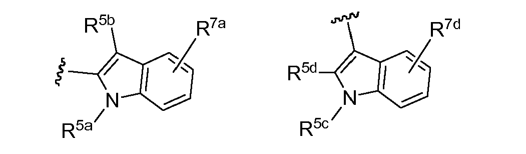 [In the formulae, the symbols are the same as above.]

Even more preferably, R⁵ and R⁵ each are a hydrogen atom or a C₁₋₆ alkyl (especially methyl); R⁵ band R^{5d} each are a C₁₋₆ alkyl (especially methyl); and R^{7a} and R^{7d} are hydrogen atoms.

Preferably, the substituents of formulae (Z-5) and (Z-6) include the following: 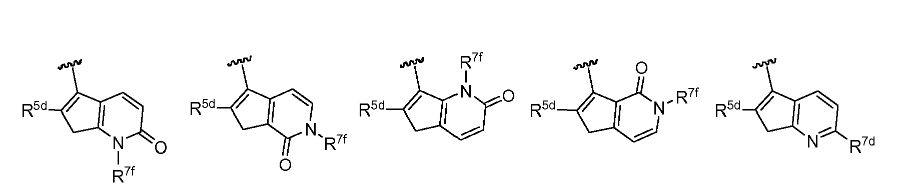 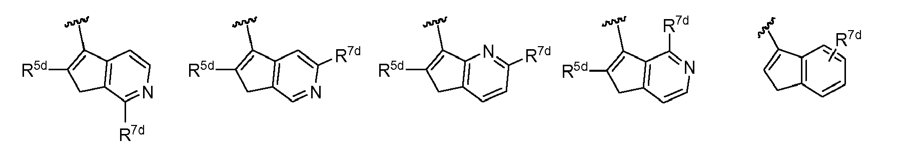 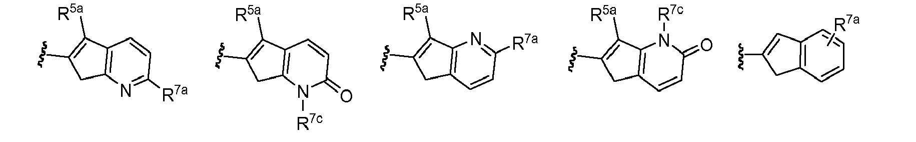 [In the formulae, the symbols are the same as above.]

Preferred examples of the compounds of formula (I) are as follows:
N-[1-(1H-indol-3-ylmethyl)-4-piperidinyl]-4-methyl-3,5-bis(methyloxy)benzamide,
4-methyl-N-{1-[(1-methyl-1-H-indol-3-yl)methyl]-4-piperidinyl}-3,5-bis(methyloxy)benzamide,
4-methyl-N-(1-{[1-methyl-5-(methyloxy)-1H-indol-3-yl]methyl}-4-piperidinyl)-3,5-bis(methyloxy)benzamide,
N- {1-[(1,2-dimethyl-1H-indol-3-yl)methyl]-4-piperidinyl}-4-methyl-3,5-bis(methyloxy)benzamide,
N-{1-[(1-ethyl-1H-indol-3-yl)methyl]-4-piperidinyl}-4-methyl-3,5-bis(methyloxy)benzamide,
4-methyl-3,5-bis(methyloxy)-N-(1-{[1-(2-propen-l-yl)-1H-indol-3-yl]methyl}-4-piperidinyl)benzamide formate,
N-(1-{[1-(difluoromethyl)-1H-indol-3-yl]methyl}-4-piperidinyl)-4-methyl-3,5-bis(methyloxy)benzamide,
4-methyl-3,5-bis(methyloxy)-N-(1-{[1-(2-propyn-1-yl)-1H-indol-3-yl]methyl}-4-piperidinyl)benzamide,
4-methyl-3,5-bis(methyloxy)-N-(1-{[5-(methyloxy)-1-propyl-1H-indol-3-yl]methyl}-4-piperidinyl)benzamide,
4-methyl-N- {1-[(3-methyl-1H-indol-2-yl)methyl]-4-piperidinyl} -3,5-bis(methyloxy)benzamide,
4-methyl-3,5-bis(methyloxy)-N-(1-{[5-(methyloxy)-1H-pyrrolo[3,2-b]pyridin-2-yl]methyl}-4-piperidinyl)benzamide,
4-methyl-N-{1-[(1-methyl-1H-indazol-3-yl)methyl]-4-piperidinyl}-3,5-bis(methyloxy)benzamide,
N-[1-(1-benzofuran-3-ylmethyl)-4-piperidinyl]-4-methyl-3,5-bis(methyloxy)benzamide,
4-methyl-3,5-bis(methyloxy)-N-[1-(thieno[2,3-b]pyridin-2-ylmethyl)-4-piperidinyl]benzamide,
4-methyl-3,5 -bis(methyloxy)-N- {1-[(4-methyl-4H-thieno[3,2-b]pyrrol-5-yl)methyl]-4-piperidinyl]benzamide.

### Methods for preparing Compounds of Formula (I)

The compounds of formula [I] may be produced, for example, according to the following production methods, to which, however, the invention should not be limited.

### Production Method 1-1:

The production method 1-1 is for obtaining a compound of formula (I) by reacting a compound of formula (II) and a compound of formula (III). 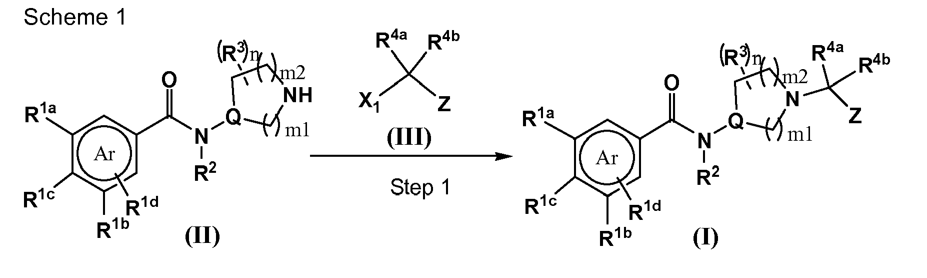 [In the formulae, X₁ represents a leaving group such as methanesulfonyloxy, benzenesulfonyloxy, p-toluenesulfonyloxy; and the other symbols are the same as above.]

### Step 1:

A compound of formula (II) is reacted with a compound of formula (III) in a reaction solvent preferably in the presence of a base to give a compound of formula (I).

The amount of the compound of formula (III) to be used may be from 1.0 to 5.0 mols relative to 1 mol of the compound of formula (II), preferably from 1.0 to 3.0 mols.

The reaction temperature may be from 25 to 100°C, preferably from 25 to 50°C, and the reaction may complete, generally from 1 to 24 hours.

The reaction is preferably in the presence of a base. For example, the base includes inorganic bases such as sodium carbonate, sodium hydrogencarbonate, potassium carbonate, potassium hydrogencarbonate, lithium carbonate; organic bases such as trimethylamine, triethylamine, diisopropylethylamine, pyridine.

The amount of the base to be used may be from 1.0 to 5.0 mols relative to 1 mol of the compound of formula (II), preferably from 1.1 to 2.0 mols.

The reaction solvent includes halogenohydrocarbons such as chloroform, methylene chloride, carbon tetrachloride; ethers such as tetrahydrofuran (hereinafter referred to as "THF), diethyl ether, 1,4-dioxane (hereinafter referred to as "dioxane"); N,N-dimethylformamide (hereinafter referred to as "DMF"), dimethyl sulfoxide (hereinafter referred to as "DMSO").

The compound of formula (II) can be prepared according to the method described below. The compound of formula (III) includes 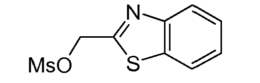 for which are usable commercially-available products and compounds derivable from commercially-available starting materials.

### Production Method 1-2:

The production method 1-2 is reductive amination of a compound of formula (II) with a compound of formula (IV) to give a compound of formula (I). 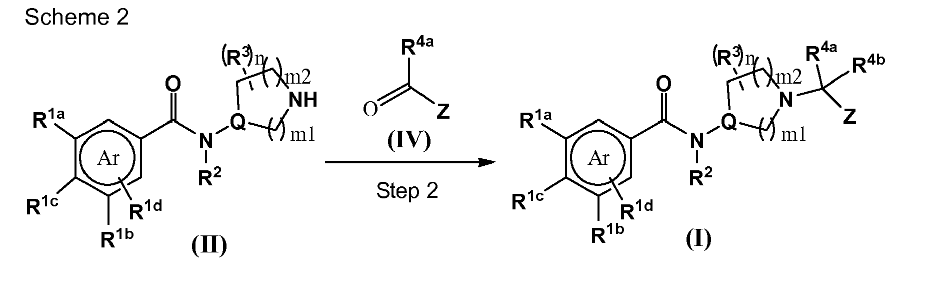 [In the formulae, the symbols are the same as above.]

### Step 2:

A compound of formula (II) is reacted with a compound of formula (IV) in a reaction solvent in the presence of a base and a reducing agent to give a compound of formula (I).

The base to be used includes, for example, triethylamine, trimethylamine, N,N-diisopropylethylamine, N-methylmorpholine, N-methylpyrrolidine, N-methylpiperidine.

The amount of the base to be used may be generally from 0 to 5 equivalents relative to 1 equivalent of the compound of the compound of formula (II), preferably from 0 to 2 equivalents.

The reducing agent to be used includes, for example, sodium cyanoborohydride, sodium triacetoxyborohydride, sodium borohydride, triethylsilane. Of those, preferred are sodium cyanoborohydride, sodium triacetoxyborohydride.

The amount of the reducing agent to be used may be generally from 1 to 20 equivalents relative to 1 equivalent of the compound of formula (II), preferably from 1 to 5 equivalents.

Not specifically limited, the reaction solvent may be any one not detracting from the reaction, and includes, for example, methanol, ethanol, chloroform, methylene chloride, 1,2-dichloroethane, THF, dioxane. Of those, preferred are methanol, methylene chloride, 1,2-dichloroethane.

If desired, an additive such as zinc(II) chloride, acetic acid, trifluoroacetic acid (hereinafter referred to as "TFA") or the like may be added to the reaction system. For example, zinc chloride may be added in an amount of 2 mols or so relative to 1 mol of the reducing agent; and acetic acid and TFA may be a catalytic amount relative to the reducing agent.

The reaction temperature may be from 0 to 100°C, preferably from 0 to 50°C, and the reaction may complete, generally from 10 minutes to 48 hours, preferably from 10 minutes to 24 hours.

The compound of formula (IV) includes the following: 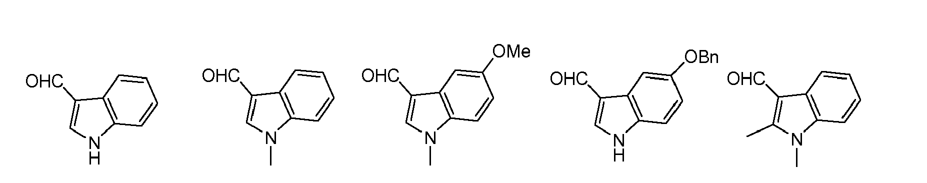 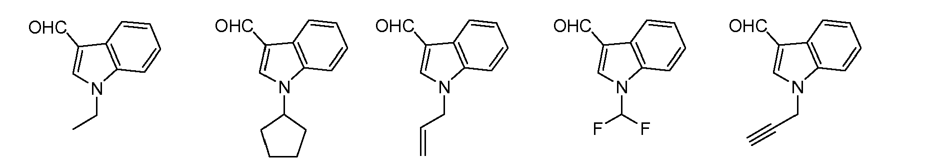 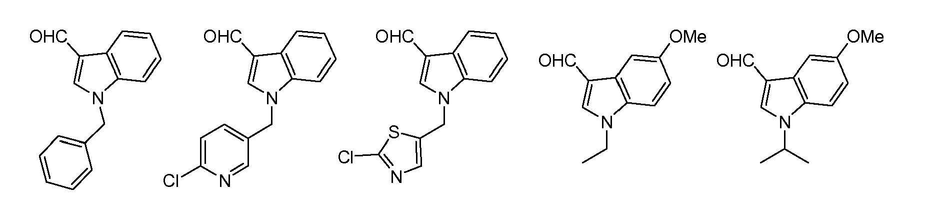 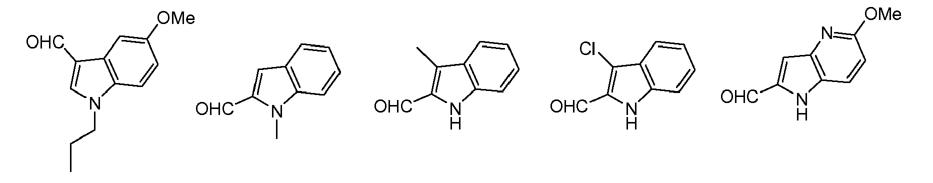 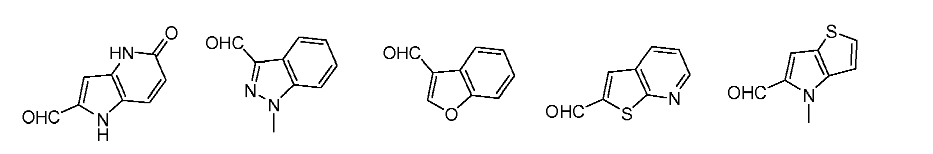 for which usable are commercially-available products.

### Production Method 1-3:

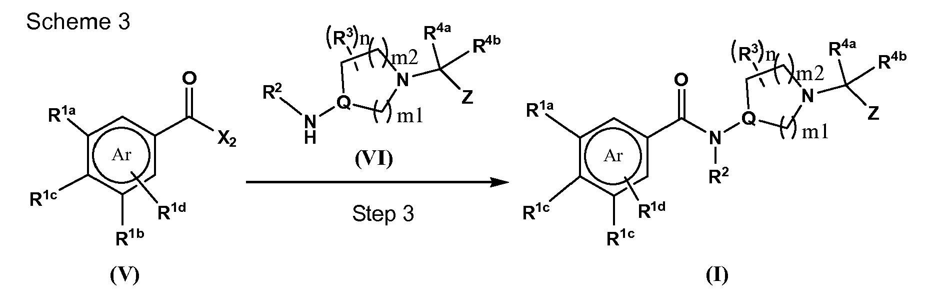

[In the formulae, X₂ represents OH, or a halogen such as Cl, F; and the other symbols are the same as above.]

### Step 3:

A compound of formula (V) is amidated with a compound of formula (VI) to give a compound of formula (I).

The amidation may be achieved by a conventional known method, including, for example, a method of reacting a compound of formula (V) and a compound of formula (VI) in the presence of a condensing agent, or a method of activating the carboxylic acid moiety of the compound of formula (V) in a conventional known manner to give a reaction equivalent, followed by amidating the equivalent with a compound of formula (VI) (for these methods, referred to is "Basis and Experiment of Peptide Synthesis" (Nobuo Izumiya, et al., Maruzen, 1983).

### 1) Method of amidation in the presence of condensing agent:

A compound of formula (V) is amidated with a compound of formula (VI), for example, in the presence or absence of N-hydroxybenzotriazole, using a condensing agent such as 1,3-dicyclohexylcarbodiimide, 1-ethyl-3-(3-dimethylaminopropyl)carbodiimide hydrochloride or the like.

The amount of the compound of formula (V) to be used may be from 0.9 to 2.0 mols relative to 1 mol of the compound of formula (VI), preferably from 1.0 to 1.5 mols.

The amount of the condensing agent to be used may be from 1.0 to 2.0 mols relative to 1 mol of the compound of formula (V), preferably from 1.0 to 1.5 mols.

The amount of HOBT, if used, may be from 0.9 to 2.0 mols relative to 1 mol of the compound of formula (V), preferably from 1.0 to 1.2 mols.

For promoting the reaction, dimethylaminopyridine may be added to the system in an amount of from 0.1 to 1.0 mol relative to 1 mol of the compound of formula (V), preferably from 0.1 to 0.5 mols.

The amidation is preferably in an organic solvent. The organic solvent includes, for example, ethers such as 1,4-dioxane, THF, diethyl ether; aromatic hydrocarbons such as benzene, toluene, xylene, chlorobenzene; halogenohydrocarbons such as dichloroethane, chloroform, dichloromethane, carbon tetrachloride; pyridine, ethyl acetate, DMF, DMSO.

The reaction temperature may be from 0 to 80°C, preferably from 20 to 50°C; and the reaction time may be from 1 to 48 hours.

### 2) Method of amidation via reaction equivalent:

A compound (carboxylic acid) of formula (V) is converted into its "reaction equivalent" according to the following method:
a) a method of converting it into an acid chloride with a chlorinating agent such as thionyl chloride, oxalyl chloride, phosphorus oxychloride or the like (acid chloride method);
b) a method of converting it into a mixed acid anhydride with isobutyl chloroformate, methyl chloroformate or the like (mixed acid anhydride method); or
c) a method of converting it into an active ester such as p-nitrophenyl ester, N-hydroxysuccinimide ester or the like (active ester method);
and thereafter the resulting "reaction equivalent" is isolated, or not isolated, and then amidated with a compound of formula (VI) to give a compound of formula (I). The "reaction equivalent" may be prepared, for example, according to the methods described in "Basis and Experiment of Peptide Synthesis" (Nobuo Izumiya, et al., Maruzen, 1983).

The amount of the reaction equivalent to be used may be from 0.8 to 3.0 mols relative to 1 mol of the compound of formula (VI), preferably from 1.1 to 1.3 mols.

The reaction may be promoted in the presence of a basic catalyst. The basic catalyst includes, for example, alkali metal carbonates such as lithium carbonate, sodium carbonate, potassium carbonate; alkali metal hydrogencarbonates such as sodium hydrogencarbonate, potassium hydrogencarbonate; organic bases such as triethylamine, diisopropylethylamine, tri-n-butylamine, 1,5-diazabicyclo[4.3.0]-5-nonene, 1,8-diazabicyclo[5.4.0]-7-undecene, pyridine, N,N-dimethylaminopyridine.

The amount of the basic catalyst to be used may be from 0.1 to 2.0 mols relative to 1 mol of the reaction equivalent, preferably from 0.1 to 1.2 mols.

As the reaction solvent, usable are those mentioned in the above. The reaction temperature may be from -50 to 80°C, preferably from 0 to 30°C. The reaction time may be from about 30 minutes to 24 hours, preferably from 30 minutes to 15 hours.

In amidation with a reaction equivalent, dimethylaminopyridine may also be used.

The compound of formula (V) includes 3,5-dimethoxy-4-methylbenzoic acid, for which are usable commercially-available products. The compound of formula (VI) may be prepared according to the method described below.

### Production Method 2:

The production method 2 is a method for preparing a compound of formula (II). 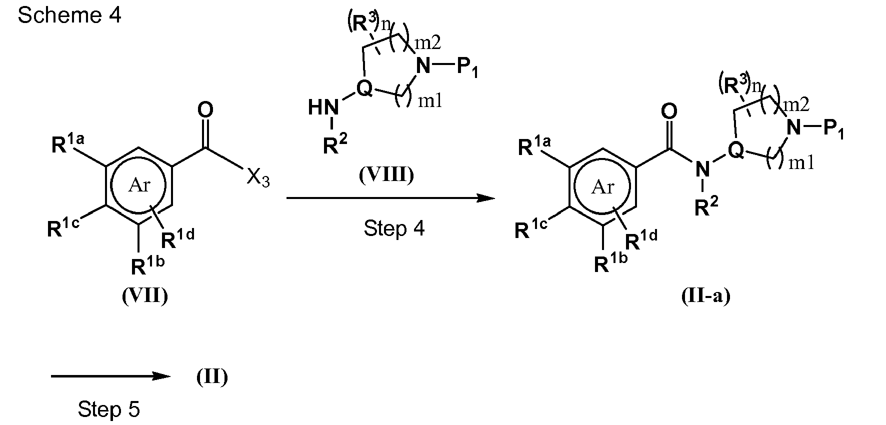 [In the formulae, P₁ represents a protective group for amino group; X₃ has the same meaning as that of X₂; and the other symbols are the same as above.]

### Step 4:

A compound of formula (VII) is amidated with a compound of formula (VIII) to give a compound of formula (II-a). The reaction condition may be the same as in the step 3.

### Step 5:

The protective group is removed from the compound of formula (II-a) to give a compound of formula (II). The deprotection may be attained according to methods described in literature (see Protective Groups in Organic Synthesis, by T. W. Greene, John Wiley & Sons, 1981), or according to methods similar thereto.

For example, in case where P₁ is a tert-butyloxycarbonyl (t-Boc) group, the compound is treated with trifluoroacetic acid, hydrochloric acid or the like at room temperature to 100°C, preferably at room temperature to 60°C for 10 minutes to 6 hours, preferably for 30 minutes to 2 hours.

The compound of formula (VII) includes 3,5-dimethoxy-4-methylbenzoic acid, for which usable are commercially-available products. The compound of formula (VIII) includes 4-amino-1-t-butyloxycarbonylpiperidine, 4-amino-1-benzylpiperidine, for which usable are commercially-available products.

### Production Method 3:

The production method 3 is a method for preparing a compound of formula (VI). 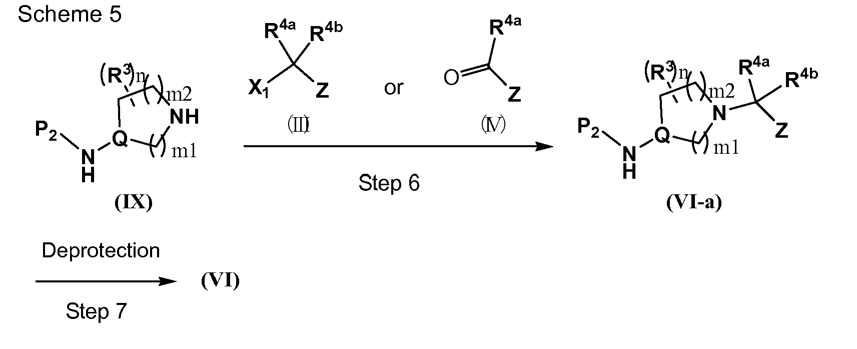 [In the formulae, P₂ has the same meaning as that of P₁; and the other symbols are the same as above.]

### Step 6:

A compound of formula (IX) is reacted with a compound of formula (III) or a compound of formula (IV) to give a compound of formula (VI-a).

The reaction condition in reacting the compound of formula (IX) with the compound of formula (III) may be the same as that in the step 1. The reaction condition in reacting the compound of formula (IX) with the compound of formula (IV) may be the same as that in the step 2.

The compound of formula (IX) includes 4-(t-butyloxycarbonylamino)-piperidine, for which usable are commercially-available products.

### Step 7:

The amino-protective group is removed from the compound of formula (VI-a) to give a compound of formula (VI). The reaction may be the same as in the step 5.

In the above-mentioned production methods where the reactant substances have an amino group, a hydroxyl group, a carboxyl group, an oxo group, a carbonyl group or the like not participating in the reaction, the amino group, the hydroxyl group, the carboxyl group, the oxo group and the carbonyl group may be suitably protected with an amino-protective group, a hydroxy-protective group, a carboxyl-protective group, or an oxo or carbonyl-protective group, then the reaction in the production method is attained, and after the reaction, the protective group may be removed.

The introduction and the removal of the protective group, through differing depending on the type of the protective group and the stability of the product compound, may be attained, for example, through solvolysis with acid or base, for example, according to methods described in literature (see Protective Groups in Organic Synthesis, T. W. Greene, John Wiley & Sons, 1981), or according to methods similar thereto, concretely, for example, according to a method of treating with from 0.01 mol to a large excessive amount of an acid, preferably trifluoroacetic acid, formic acid, hydrochloric acid or the like, or with from an equimolar amount to a large excessive amount of a base, preferably potassium hydroxide, calcium hydroxide or the like; or through chemical reduction with a metal hydride complex or the like, or through catalytic reduction with a palladium-carbon catalyst, a Raney-nickel catalyst or the like.

Not specifically limited, the amino-protective group may be any one having its function, and includes, for example, an aralkyl such as benzyl, p-methoxybenzyl, etc.; a C₁₋₆ alkanoyl such as acetyl, propionyl, etc.; benzoyl; an arylalkanoyl such as phenylacetyl, etc.; a C₁₋₆ alkyloxycarbonyl such as methoxycarbonyl, ethoxycarbonyl, tert-butoxycarbonyl, etc.; an alkyloxycarbonyl such as benzyloxycarbonyl, p-nitrobenzyloxycarbonyl, etc.; a C₁₋₆ alkylsilyl such as trimethylsilyl, tert-butyldimethylsilyl, etc.; tetrahydropyranyl; trimethylsilylethoxymethyl; a C₁₋₆ alkylsulfonyl such as methylsulfonyl, ethylsulfonyl, etc; an arylsulfonyl such as benzenesulfonyl, toluenesulfonyl, etc. Especially preferred are acetyl, benzoyl, tert-butoxycarbonyl, trimethylsilylethoxymethyl, methylsulfonyl, etc.

Not specifically limited, the hydroxyl-protective group may be any one having its function, and includes, for example, a C₁₋₆ alkyl such as methyl, ethyl, propyl, etc.; a C₁₋₆ alkylsilyl such as trimethylsilyl, tert-butyldimethylsilyl, etc.; a C₁₋₆ alkyloxymethyl such as methoxymethyl, 2-methoxyethoxymethyl, etc.; tetrahydropyranyl; trimethylsilylethoxymethyl; an aralkyl such as benzyl, p-methoxybenzyl, trityl, etc.; an acyl such as formyl, acetyl, etc. Especially preferred are methyl, methoxymethyl, tetrahydropyranyl, trityl, trimethylsilylethoxymethyl, tert-butyldimethylsilyl, acetyl, etc.

Not specifically limited, the carboxyl-protective group may be any one having its function, and includes, for example, a C₁₋₆ alkyl such as methyl, ethyl, propyl, tert-butyl, etc.; a halo-C₁₋₆ alkyl such as 2,2,2-trichloroethyl, etc.; a C₂₋₆ alkenyl such as 2-propenyl, etc.; an aralkyl such as benzyl, p-methoxybenzyl, p-nitrobenzyl, trityl, etc. Especially preferred are methyl, ethyl, tert-butyl, 2-propenyl, benzyl, p-methoxybenzyl, etc.

Not specifically limited, the oxo or carbonyl-protective group may be any one having its function, and includes, for example, acetals and ketals such as ethylene ketal, dimethyl ketal, S,S'-dimethyl ketal, etc.

The compounds of formula (I) obtained in the manner as above may be readily isolated and purified in any ordinary separation method of, for example, solvent extraction, recrystallization, column chromatography, preparative thin-layer chromatography or the like.

The effect of the compounds of the invention as an MCH receptor antagonist is shown, for example, by the following pharmacological test example.

### Pharmacological Test Example: MCH binding inhibition test

A human MCH-1R encoding cDNA sequence [FEBS Letters, Vol. 398, 253 (1996); Biochimica et Biophisica Acta, Vol. 1401, 216 (1998)] was cloned to a plasmid vector pEF/myc/cyto (by Invitrogen Corporation). The obtained expression vector was transfected to host cells CHO-K1 (American Type Culture Collection) using Lipofectamine Plus Reagent (by Life Technology Inc.) to provide MCH-1R expression cells.

Membrane samples prepared from the MCH-1R expression cells were incubated with each test compound and 50 pM of [¹²⁵I]MCH (by NEN Co.), in an assay buffer (50 mM Tris buffer comprising 10 mM magnesium chloride, 2 mM ethylenediamine tetraacetate, 0.01% bacitracin and 0.2 % bovine serum albumin; pH 7.4) at 25°C for an hour, followed by filtration through a glass filter GF/C (by Wattman Co.). After washing the glass filter with 50 mM Tris buffer (pH 7.4) comprising 10 mM magnesium chloride, 2 mM ethylenediamine tetraacettic acid and 0.04 % Tween-20, the radio activity on the glass filter was measured. The non-specific binding was measured in the presence of 1 µM human MCH, and the 50 % inhibition concentration (IC₅₀ value) of each test compound to the specific [¹²⁵I]MCH binding was determined. The results are shown in Table 1.

**Table 1**

| Example | Structure | **IC50(nM)** |
|---|---|---|
| **2-2** | 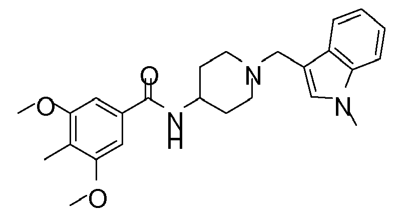 | **13** |
| **2-5** | 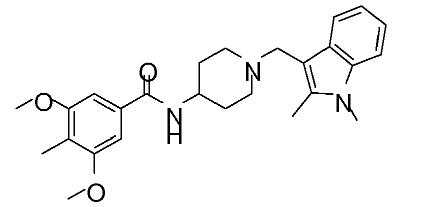 | **26** |
| **2-10** | 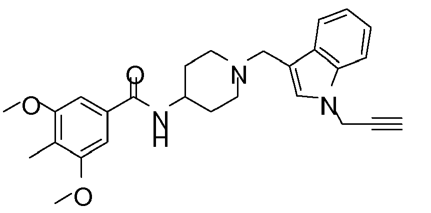 | **22** |
| **2-18** | 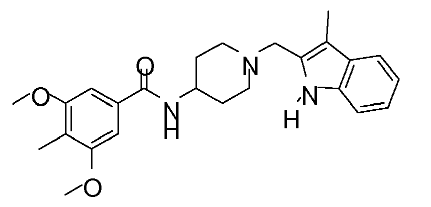 | **6.8** |
| **2-25** | 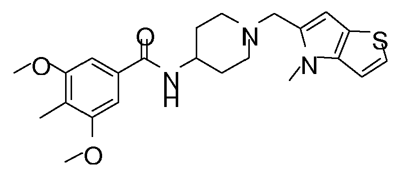 | **16** |

As in the above, the compounds of the invention strongly inhibit the binding of MCH to MCH-1R, and therefore exhibit an excellent effect as an MCH-IR antagonist.

Accordingly, the compounds of the invention are useful as a preventive or a remedy for various MCH-related diseases, for example, metabolic disorders such as obesity, diabetes, hormone disorder, hyperlipidemia, gout, fatty liver, etc.; cardiovascular disorders such as stenocardia, acute/congestive heart failure, myocardial infarction, coronary atherosclerosis, renal diseases, electrolyte abnormality, etc.; central or peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism, etc.; reproductive disorders such as infertility, preterm labor, sexual dysfunction, etc.; and other digestive disorders; respiratory disorders; cancer or pigmentation; especially as a preventive or a remedy for obesity, diabetes, fatty liver, bulimia, depression, anxiety.

### Pharmaceutical Composition Comprising Compound of Formula [I]

The compound of the invention can be orally or parenterally administered, and can be formulated into preparations suitable to the administration thereof, which may be used as pharmaceutical compositions for prevention, treatment or therapy for the above-mentioned diseases.

In its clinical use, the compound of the invention may be formulated into various preparations along with a pharmaceutically-acceptable carrier added thereto generally in accordance with the administration route thereof, and the thus-formulated pharmaceutical composition may be administered. Various ordinary additives used in the field of pharmaceutical preparations can be used. For example, they include gelatin, lactose, white sugar, titanium oxide, starch, crystalline cellulose, hydroxypropylmethyl cellulose, carboxymethyl cellulose, corn starch, microcrystalline wax, white petrolatum, magnesium aluminate metasilicate, anhydrous calcium phosphate, citric acid, trisodium citrate, hydroxypropyl cellulose, sorbitol, sorbitan fatty acid esters, polysorbate, sucrose fatty acid esters, polyoxyethylene, hardened castor oil, polyvinylpyrrolidone, magnesium stearate, light silicic anhydride, talc, vegetable oils, benzyl alcohol, gum arabic, propylene glycol, polyalkylene glycol, cyclodextrin, hydroxypropylcyclodextrin, etc.

Preparations to be formed with those additives include, for example, solid preparations such as tablets, capsules, granules, powders, suppositories, etc.; and liquid preparations such as syrups, elixirs, injections, etc. These may be formulated according to ordinary methods known in the field of pharmaceutical preparations. The liquid preparations may also be in such a form that may be dissolved or suspended in water or in any other suitable medium in their use. Especially for injections, if desired, the preparations may be dissolved or suspended in physiological saline water or glucose liquid, and a buffer or a preservative may be optionally added thereto.

The pharmaceutical compositions may contain the compound of the invention in an amount of from 1 to 99.9 % by weight, preferably from 1 to 60 % by weight of the composition. The compositions may further contain any other therapeutically-effective compound.

In case where the compounds of the invention are used for prevention, treatment or remedying for the above-mentioned diseases, then the dose and the dosing frequency may be varied, depending on the sex, the age, the body weight and the disease condition of the patient, on the type and the range of the intended remedial effect, etc. In general, in oral administration, the dose may be from 0.001 to 50 mg/kg of body weight/day, and it may be administered at a time or in a few times. The dose is preferably from about 0.01 to about 25 mg/kg/day, more preferably from about 0.05 to about 10 mg/kg/day.

As combination therapy, the compounds of the invention can be used in combination with drugs effective for hypertension, obesity-associated hypertension, hypertension-associated diseases, hypertrophy, left ventricular hypertrophy, metabolic disorders, obesity, obesity-associated diseases and the like (hereafter referred to as "co-drugs"). Such drugs can be administered simultaneously, separately or in succession, for prevention or treatment of the above-mentioned diseases. When a compound of the invention is used simultaneously with one, two or more of co-drugs, they may be formulated into a medical preparation suited for single administration form. Whereas, in combination therapy, a composition containing the compound of the invention and co-drugs may be administered to the object of medication in different packages, either simultaneously, separately or successively. They may be administered at time intervals.

The dose of the co-drug may be determined in accordance with the clinically adopted dose thereof, which can be suitably selected according to the individual object of medication, the administration route, the specific disease, the combination of drugs, and the like. The form of the co-drug for administration is not specifically defined, and it may be combined with the compound of the invention when they are administered.

The administration mode includes, for example, the following: (1) A compound of the invention is combined with a co-drug to give a single preparation for single administration; (2) a compound of the invention and a co-drug are separately formulated into different two preparations, and the two preparations are simultaneously administered in one administration route; (3) a compound of the invention and a co-drug are separately formulated into different two preparations, and they are administered at different times in one and the same administration route; (4) a compound of the invention and a co-drug are separately formulated into different two preparations, and they are administered at the same time in two different administration routes; (5) a compound of the invention and a co-drug are separately formulated into different two preparations, and they are administered at different times in different administration routes (for example, a compound of the invention and a co-drug are administered in that order, or in an order contrary to this). The blend ratio of the compound of the invention and the co-drug may be suitably determined depending on the administration object, the administration route, and the disease for the administration.

The co-drugs usable in the invention include, for example, drugs for diabetes, drugs for hyperlipidemia, drugs for hypertension, anti-obesity drugs. Two or more such co-drugs may be combined in an adequate ratio and used.

The remedy for diabetes include, for example, 1) PPAR-γ agonists such as glitazones (e.g., ciglitazone, darglitazone, englitazone, isaglitazone (MCC-555) et al), pioglitazone, rosiglitazone, troglitazone, BRL49653, CLX-0921, 5-BTZD, GW-0207, LG-100641, LY-300512et al; 2) biguanides such as metformin, buformin, phenformin et al; 3) protein tyrosine phosphatase 1B inhibitors; 4) sulfonylureas such as acetohexamide, chloropropamide, diabinese, glibenclamide, glipizide, glyburide, glimepiride, gliclazide, glipentide, gliquidone, glisolamide, trazamide, tolubutamide et al; 5) meglitinides such as repaglinide, nateglinide et al; 6) α-glucoside hydroxylase inhibitors such as acarbose, adiposine, camiglibose, emiglitate, miglitol, voglibose, pradimicin-Q, salbostatin, CKD-711, MDL-25, 673, MDL-73, 945, MOR14 et al; 7) α-amylase inhibitors such as tendamistat, trestatin, A13688 et al; 8) insulin secretion promoters such as linogliride, A-4166 et al; 9) fatty acid oxidation inhibitors such as clomoxir, etomoxir et al; 10) A2 antagonists such as midaglizole, isaglidole, deriglidole, idazoxan, earoxan, fluparoxan et al; 11) insulin or insulin mimetics such as biota, LP-100, novalapid, insulindetermir, insulin lispro, insulin glargine, insulin zinc, Lys-Pro-insulin, GLP-1 (73-7), GLP1 amide (7-36) et al; 12) non-thiazolidinediones such as JT-501, farglitazar et al; 13) PPARα/γ dual-agonists such as MK-0767, CLX-0940, GW-1536, GW-1929, GW-2433, KRP-297, L-796449, LR-90 and SB219994 et al.

The remedy for hyperlipidemia include, for example, 1) bile acid absorption promoters such as cholesterylamine, colesevelem, colestipol, crosslinked dextran dialkylaminoalkyl derivatives, Colestid^{™}, LoCholest^{™}, Questran^{™} et al; 2) HMG-CoA reductase inhibitors such as atorvastatin, itavastatin, fluvastatin, lovastatin, pravastatin, rivastatin, rosuvastatin, simvastatin, ZD-4522 et al; 3) HMG-CoA synthase inhibitors; 4) cholesterol absorption inhibitors such as snatol ester, β-sitosterol, sterol glucoside, ezetimibe et al; 5) acyl-coenzyme A·cholesterol acyl transferase inhibitors such as avasimibe, eflucimibe, KY-505, SMP-709 et al; 6) CETP inhibitors such as JTT705, torcetrapib, CP532632, BAY-63-2149, SC-591, SC-795 et al; 7) squalane synthesis inhibitors; 8) antioxidants such as probucol; 9) PPAR-α agonists such as beclofibrate, benzafibrate, ciprofibrate, clofibrate, etofibrate, fenofibrate, gemcabene, gemfibrozil, GW-7647, BM-170744, LY-518674, fibric acid derivatives (e.g., Atromid^{™}, Lopid^{™}, Tricor^{™}) et al; 10) FXR receptor antagonists such as GW-4064, SR-103912 et al; 11) LXR receptor agonists such as GW3965, T9013137, XTCO-179628 et al; 12) lipoprotein synthesis inhibitors such as niacin; 13) renin-angiotensin system inhibitors; 14) microsome-triglyceride transport inhibitors; 15) bile acid resorption inhibitors such as BARA1453, SC435, PHA384640, S-435, AZD7706 et al; 16) PPAR-δ agonists such as GW501516, GW590735 et al; 17) triglyceride synthesis inhibitors; 18) MTTP inhibitors such as LAB687, CP346086 et al; 19) low-density lipoprotein receptor inducers; 20) squalane epoxidase inhibitors; 21) platelet agglutination inhibitors; 22) 5-lipoxygenase activated protein inhibitors such as MK-591.

The remedy for hypertension include, for example, 1) thiazide diuretics such as chlorothialidon, chlorothiazide, dichlorofenamide, hydrofluorothiazide, indapamide, hydrochlorothiazide et al; loop diuretics such as bumetanide, ethacrynic acid, flosemide, tolusemide et al; sodium diuretics such as amyloride, triamterene et al; aldosterone antagonist diuretics such as spironolactone, epilenone et al; 2) β-adrenaline blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, probanolol, sotalol, tertatolol, tilisolol, timolol et al; 3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, verapamil et al; 4) angiotensin converting enzyme inhibitors such as benazepril, captopril, cilazapril, delapril, enalapril, fosinopril, imidapril, rosinopril, moexipril, quinapril, quinaprilat, ramipril, perindopril, perindoropril, quanipril, spirapril, tenocapril, trandolapril, zofenopril et al; 5) neutral endopeptidase inhibitors such as omapatrilat, cadoxatril, ecadotril, fosidotril, sampatrilat, AVE7688, ER4030 et al; 6) endotheline antagonists such as tezosentan, A308165, YM62899 et al; 7) vasodilators such as hydralazine, clonidine, minoxidil, nicotinyl alcohol et al; 8) angiotensin II antagonists such as candesartan, eporsartan, iribesartan, losartan, pratosartan, tasosartan, telmisartan, valsartan, EXP-3137, FI6828K, RNH6270 et al; 9) α/β adrenaline blockers such as nipradilol, arotinolol, amoslalol et al; 10) α1 blockers such as terazosin, urapidil, prazosin, bunazosin, trimazosin, doxazosin, naphthopidil, indolamin, WHIP164, XEN010 et al; 11) α2 agonists such as lofexidine, tiamenidine, moxonidine, rilmenidine, guanobenz et al; and 12) aldosterone inhibitors.

The anti-obesity drugs include, for example, 1) 5HT (serotonin) transporter inhibitors such as paroxetine, fluoxetine, fenfluramine, fluvoxamine, sertraline, imipramine et al; 2) norepinephrine transporter inhibitors such as GW320659, desipramine, talsupram, nomifensin et al; 3) cannabinoid-1 receptor 1 (CB-1) antagonists/inverse-agonists such as limonabant (Sanofi Synthelabo), SR-147778 (Sanofi Synthelabo), BAY-65-2520 (Bayer), SLV-319 (Solvey), as well as compounds disclosed in USP 5,532,237, USP 4,973,587, USP 5,013,837, USP 5,081,122, USP 5,112,820, USP 5,292,736, USP 5,624,941, USP 6,028,084, WO96/33159, WO98/33765, WO98/43636, WO98/43635, WO01/09120, WO01/96330, WO98/31227, WO98/41519, WO98/37061, WO00/10967, WO00/10968, WO97/29079, WO99/02499, WO01/58869, WO02/076949, WO01/64632, WO01/64633, WO01/64634, WO03/006007, WO03/007887 and EP-658546 et al; 4) ghrelin antagonists such as compounds disclosed in WO01/87355, WO02/08250 et al; 5) histamine(H3) antagonists/inverse-agonists such as thioperamide, 3-(1H-imidazol-4-yl)propyl N-(pentenyl)carbonate, clobenpropit, iodofenpropit, imoproxyfen, GT2395, A331440, compounds disclosed in WO02/15905, O-[3-(1H-imidazol-4-yl)propanol] carbamate, piperazine-containing H3-receptor antagonists (Lazewska, D. et al., Pharmazie, 56: 927-32 (2001), benzophenone derivatives (Sasse, A. et al., Arch. Pharm. (Weinheim) 334: 45-52 (2001)), substituted N-phenylcarbamates (Reidemeister, S. et al., Pharmazie, 55: 83-6 (2000)), proxyfen derivatives (Sasse, A. et al., J. Med. Chem., 43: 3335-43 (2000)) et al; 6) MCH-1R antagonists such as T-226296 (Takeda), SNP-7941 (Synaptic), other compounds disclosed in WO01/82925, WO01/87834, WO02/051809, WO02/06245, WO02/076929, WO02/076947, WO02/04433, WO02/51809, WO02/083134, WO02/094799, WO03/004027 and JP-A-2001-226269 et al; 7) MCH-2R agonists/antagonists; 8) NPY1 antagonists such as isopropyl 3-chloro-5-(1-(6-[2-(5-ethyl-4-methyl-thiazol-2-yl)-ethyl]-4-morpholinyl-4-yl-piridin-2-ylamino)-ethyl)phenyl]carbamate, BIBP3226, BIBO3304, LY-357897, CP-671906, GI-264879, and other compounds disclosed in USP 6,001,836, WO96/14307, WO01/23387, WO99/51600, WO01/85690, WO01/85098, WO01/85173 and WO01/89528 et al; 9) NPY5 antagonists such as 152804, GW-569180A, GW-594884A, GW-587081X, GW-548118X, FR235,208, FR226928, FR240662, FR252384, 1229U91, GI-264879A, CGP71683A, LY-377897, LY366377, PD-160170, SR-120562A, SR-120819A, JCF-104, H409/22, and other compounds disclosed in USP 6,140,354, USP 6,191,160, USP 6,258,837, USP 6,313,298, USP 6,337,332, USP 6,329,395, USP 340,683, USP 6,326,375, USP 6,329,395, USP 6,337,332, USP 6,335,345, EP-01010691, EP-01044970, WO97/19682, WO97/20820, WO97/20821, WO97/20822, WO97/20823, WO98/27063, WO00/107409, WO00/185714, WO00/185730, WO00/64880, WO00/68197, WO00/69849, WO01/09120, WO01/14376, WO01/85714, WO1/85730, WO01/07409, WO01/02379, WO01/02379, WO01/23388, WO01/23389, WO01/44201, WO01/62737, WO01/62738, WO01/09120, WO02/20488, WO02/22592, WO02/48152, WO02/49648, WO02/094789, and compounds disclosed in Norman et al., J. Med. Chem., 43:4288-4312(2000) et al; 10) leptins such as human recombinant leptin (PEG-OB, Hoffman La Roche), recombinant methionylleptin (Amgen) et al; 11) leptin derivatives such as compounds disclosed in USP 5,552,524, USP 5,552,523, USP 5,552,522, USP 5,521,283, WO96/23513, WO96/23514, WO96/23515, WO96/23516, WO96/23517, 96/23518, WO96/23519 and WO96/23520 et al; 12) opioid antagonists such as nalmefen (Revex^{™}), 3-methoxynaltorexone, naloxone, naltorexone, compounds disclosed in WO00/21509 et al; 13) orexin antagonists such as SB-334867A, and other compounds disclosed in WO01/96302, WO01/68609, WO02/51232, WO02/51838 and WO03/023561 et al; 14) bombesin receptor subtype-3 agonists; 15) cholecystokinin A (CCK-A) agonists such as AR-R15849, GI-181771, JMV-180, A-71378, A-71623, SR-146131, and other compounds disclosed in USP 5,739,106 et al; 16) CNTF (ciliary neurotrophic factors) such as GI-181771 (Glaxo-Smith Kline), SR146131 (Sanofi Synthelabo), butabindide, PD170,292, PD149164 (Pfizer) et al; 17) CNTF derivatives such as axokine (Regeneron), and other compounds disclosed in WO94/09134, WO98/22128, WO99/438 et al; 18) growth hormone secretion receptor agonists such as NN703, hexarelin, MK-0677, SM-130686, CP-424,391, L-692,429, L-163,255, and compounds disclosed in USP 6,358,951, US Patent Application Nos. 2002/049196, 2002/022637, WO01/56592, WO02/32888 et al; 19) serotonin receptor-2C agonists such as BVT933, DPCA37215, IK264, PNU22394, WAY161503, R-1065, YM348, and other compounds disclosed in USP 3,914,250, WO02/36596, WO02/48124, WO02/10169, WO01/66548, WO02/44152, WO02/51844, WO02/40456 and WO02/40457 et al; 20) melanocortin-3 receptor agonists; 21) melanocortin-4 receptor agonists such as CHIR86036 (Chiron), ME-10142, ME-10145 (Melacure), and other compounds disclosed in WO99/64002, WO00/74679, WO01/991752, WO01/74844, WO01/70708, WO01/70337, WO01/91752, WO02/059095, WO02/059107, WO02/059108, WO02/059117, WO02/12166, WO02/11715, WO02/12178, WO02/15909, WO02/068387, WO02/068388, WO02/067869, WO03/007949 and WO03/009847 et al; 22) monoamine resorption inhibitors such as sibutramine (Meridia^{™}/Reductil^{™}) and its salts, and other derivatives disclosed in USP 4,746,680, USP 4,806,570, USP 5,436,272, US Patent Application No. 2002/0006964, WO01/27068 and WO01/62341 et al; 23) serotonin re-uptake inhibitors such as dexfenfluramine, fluoxetine, and other compounds disclosed in USP 6,365,633, WO01/27060 and WO01/162341 et al; 24) glucagon-like peptide-1 agonists; 25) topiramate (Topimax^{™}); 26) phytopharm compound 57 (e.g., CP644,673); 27) acetyl CoA carboxylase-2 (ACC2) inhibitors; 28) β-adrenalin receptor-3 agonists such as AD9677/TAK677 (Dai-Nippon Pharmaceutical/Takeda Chemical), CL-316,243, SB418790, BRL-37344, L-796568, BMS-196085, BRL-35135A, CGP12177A, BTA-243, W427353, Trecadrine, Zeneca D7114, SR59119A, and other compounds disclosed in USP 5,705,515, USP 5,451,677, WO01/74782 and WO02/32897 et al; 29) diacylglycerol acyltransferase-1 inhibitors; 30) diacylglycerol acyltransferase-2 inhibitors, 31) fatty acid synthesis inhibitors such as carulenin, C75; 32) phosphodiesterase inhibitors such as theophylline, pentoxiphylline zaprinast, sildenafil, amrinone, milrinone, cilostamide, rolipram and cilomilast et al; 33) thyroid hormone-β agonists such as KB-2611 (KaroBio BMS), and other compounds disclosed in WO02/15845, JP-A-2000-256190 et al; 34) UCP (uncoupling protein)-1, 2, or 3 activators such as phytanic acid, 4-[(E)-2-(5,6,7,8-tetrahydro-5,5,8,8-tetramethyl-2-naphthalenyl)-1-propenyl]benzoic acid (TTNPB), retinoic acid, and other compounds disclosed in WO99/00123 et al; 35) acylestrogens such as oleoylestrone, and other compounds disclosed in del Mar-Grasa, M. et al., Obesity Research, 9:202-9 (2001) et al; 36) glucocorticoid antagonists; 37) 11-β-hydroxysteroid dehydrogenase-1 inhibitors such as BVT3498, BVT2733, and other compounds disclosed in WO01/90091, WO01/90090, WO01/90092 et al; 38) stearoyl-CoA desaturase-1 inhibitors; 39) dipeptidyl peptidase-IV inhibitors such as isoleucine thiazolidide, valine pyrrolidide, NVP-DPP728, AF237, P93/01, TSL225, TMC-2A/2B/2C, FE999011, P9310/K364, VIP0177, SDZ274-444, and other compounds disclosed in WO03/004498, WO03/004496, EP1258476, WO02/083128, WO02/062764, WO03/000250, WO03/002530, WO03/002531, WO03/002553, WO03/002593, WO03/000180 and WO03/0001 et al; 40) lipase inhibitors such as tetrahydroliptatin (Orlistat/Xenical^{™}), Triton WR1339, RHC80267, lipstatin, teasaponin, diethylumbelliferyl phosphate, FL-386, WAY-121898, Bay-N-3176, valilactone, esteracin, ebelactone A, ebelactone B, RHC80267, and other compounds disclosed in WO01/77094, USP 4,598,089, USP 4,452,813, USP 5,512,565, USP 5,391,571, USP 5,602,151, USP 4,405,644, USP 4,189,438 and USP 4,242,453 et al; 41) fatty acid transporter inhibitors; 42) dicarboxylate transporter inhibitors; 43) glucose transporter inhibitors; 44) phosphate transporter inhibitors.

Those combined drugs are obtained by combining a compound of the invention with one, two or more of the above co-drugs. Furthermore, the combined drugs are useful for prevention or treatment of metabolic disorders, when combined with one, two or more drugs selected from the group consisting of remedy for diabetes and remedy for hyperlipidemia. Combinations containing, in particular, remedy for hypertension and anti-obesity agent are useful for prevention or treatment of metabolic disorders with synergistic effect, when remedy for diabetes and/or remedy for hyperlipidemia are added thereto.

On the other hand, the compound of the invention may be combined with an antipsychotic. An antipsychotic, especially an atypical antipsychotic is known to have a side effect of body weight increase; and the compound of the invention, when combined with such an antipsychotic, is useful for retarding the side effect. The antipsychotic includes, for example, olanzapine, Risperidone, quetiapine, Ziprasidone, aripiprazole, Paliperidone, Clozapine et al. Using an antipsychotic, as combined with a compound of the invention, may improve the level of metabolic parameters such as the level of blood pressure, glucose and lipid level that may be elevated by the antipsychotic. The above-mentioned methods may apply to the conditions of dose, administration subj ect, administration route and administration form.

### BEST MODE FOR CARRYING OUT THE INVENTION

The invention is described more concretely with reference to the following Examples, to which, however, the invention should not be limited. The compounds of the invention may be produced, for example, according to a combination of the method described in Bioorganic Medicinal Chemistry Letters, Vol. 15, pp. 4174-4179, 2005, and a known technique. As silica gel for column, used was Wakogel^{™} C-200 (Wako Pure Chemical Industries); as a filled silica gel column, used was FLASH+^{™} cartridge, KP-Sil or FPNH, FLASH12+M, FLASH25+S, FLASH25+M, FLASH40+M (Biotage Japan), TC-C18 (Agilent), Extend-C18 (Zorbax); and as a partitioning thin-layer chromatogram, used was Kieselgel 60F254 (Merck). For ¹H-NMR, used was JNM-AL400 (JEOL) or MERCURYvx400 (VARIAN) and ^{UNITY}INOVA400 (VARIAN); and for mass spectrometry, used was ZQ2000 (Waters).

### EXAMPLES

### Reference Example 1 - Production of compound (IIa):

### Reference Example 1-1:

### Production of 1,1-dimethylethyl 4-({[4-methyl-3,5-bis(methyloxy)phenyl]carbonyl}amino)-1-piperidinecarboxylate

At 0°C, thionyl chloride (7.44 mL) was added to 3,5-dimethoxy-4-methylbenzoic acid (2.00 g), and stirred at 50°C for 1 hour. Toluene was added to the reaction solution, and thionyl chloride was removed through azeotropy. The resulting black solid residue was dissolved in chloroform (5.0 mL), to which was added a chloroform solution (5.0 mL) of 4-amino-Boc-piperidine (2.04 g) at 0°C, and stirred at room temperature for 1 hour. Aqueous saturated sodium hydrogencarbonate solution was added to the reaction liquid, and this was extracted with chloroform. The organic layer was washed with saturated brine, and dried with sodium sulfate. This was concentrated under reduced pressure, and hexane was added to the brown solid residue and irradiated with ultrasonic waves. The reaction mixture was filtered, and washed (hexane). This was dried under reduced pressure to give the entitled compound (3.71 g) as a light brown solid.
ESI-MS Found: m/z 379[M+H]⁺

### Reference Example 1-2:

### Production of 4-methyl-3,5-bis(methyloxy)-N-[1-(phenylmethyl)-4-piperidinyl]benzamide

Pyridine (12 mL) was added to 3,5-dimethoxy-4-methylbenzoic acid (1.00 g), 4-amino-1-benzylpiperidine (1.0 g) and 1-ethyl-3-(3'-dimethylaminopropyl)carbodiimide hydrochloride (0.98 g), and stirred for one full day at 60°C. The reaction liquid was cooled, then poured into water (200 mL), and stirred at room temperature for 30 minutes. The precipitated solid was collected through filtration, and washed with water. The resulting solid was dried at 40°C under reduced pressure to give the entitled compound (1.24 g) as a brown solid. ¹H-NMR (400 MHz, CDC13 , δ ppm) : 1.53-1.65 (2H, m), 2.00-2.08 (2H, m), 2.11 (3H, s), 2.14-2.25 (2H, m), 2.83-2.90 (2H, m), 3.53 (2H, 2), 3.86 (6H, s), 3.95-4.04 (1H, m), 5.90 (1H, d, J = 7.2 Hz), 6.87 (2H, s), 7.30-7.35 (5H, m).
ESI-MS Found : m/z 369[M+H]⁺

### Reference Example 2 - Production of compound (II):

### Reference Example 2-1:

### Production of 4-methyl-3,5-bis(methyloxy)-N-4-piperidinylbenzamide hydrochloride

At 0°C, 4 N hydrogen chloride/1,4-dioxane solution (3.0 mL) was added to the compound (500 mg) obtained in Reference Example 1-1, and stirred at room temperature for 1 hour. The reaction liquid was azeotroped with toluene, then hexane was added to the residue and irradiated with ultrasonic waves. The resulting solid was filtered, washed (hexane), and dried under reduced pressure to give the entitled compound (396 mg) as a brown solid.
ESI-MS Found: m/z 279[M+H]⁺

### Reference Example 2-2:

### Production of 4-methyl-3,5-bis(methyloxy)-N-4-piperidinylbenzamide

20 % palladium hydroxide/carbon (200 mg) was added to a methanol solution (400 mL) of the compound (500 mg) obtained in Reference Example 1-2, and stirred in a hydrogen atmosphere at room temperature for 18 hours. The reaction liquid was filtered through Celite, and the filtrate was concentrated under reduced pressure to give the entitled compound (370 mg) as a white solid.
ESI-MS Found : m/z 279[M+H]⁺

### Example 1:

### Example 1-1:

### Production of N-[1-(1,3-benzothiazol-2-ylmethyl)-4-piperidinyl]-4-methyl-3,5-bis(methyloxy)benzamide:

At 0°C, methanesulfonyl chloride (47 µL) was added to an ethyl acetate solution (2.0 mL) of 1,3-benzothiazol-2-ylmethanol (40.1 mg) and diisopropylethylamine (127 µL), and stirred at 0°C for 20 minutes. The reaction liquid was poured into aqueous sodium hydrogencarbonate solution to stop the reaction, and then extracted with ethyl acetate. The organic layer was dried with sodium sulfate, and concentrated under reduced pressure to give a crude product of 1,3-benzothiazol-2-ylmethyl methanesulfonate as a yellow oily substance. At 0°C, a chloroform solution (1.5 mL) of the compound was added to chloroform solution (1.0 mL) of the compound (76.0 mg) obtained in Reference Example 2-1 and diisopropylethylamine (210 µL), and stirred at 0°C for 1 hour and then stirred at room temperature for 14 hours. Aqueous sodium hydrogen carbonate solution was added to the reaction liquid, and extracted with chloroform. The organic layer was dried with sodium sulfate, and concentrated under reduced pressure. The resulting residue was purified through preparative thin-layer chromatography (chloroform/methanol = 95/5) to give the entitled compound (74.4 mg) as a white solid.
¹H-NMR (400 MHz, CDCl₃ ,δ ppm) : 1.62-1.72 (2H, m), 2.07-2.11 (2H, m), 2.11 (3H, s), 2.41-2.48 (2H, m), 3.00-3.03 (2H, m), 3.87 (6H, s), 3.97 (2H, s), 4.00-4.10 (1H, m), 6.01 (1H, d, J = 8.0 Hz), 6.90 (2H, s), 7.35-7.39 (1H, m), 7.44-7.48 (1H, m), 7.86-7.88 (1H, m), 7.96-7.99 (1H, m).
ESI-MS Found : m/z 426[M+H]⁺

### Example 2:

### Example 2-1:

### Production of N-[1-(1H-indol-3-ylmethyl)-4-piperidinyl]-4-methyl-3,5-bis(methyloxy)benzamide

Indole-3-aldehyde (68 mg) and sodium triacetoxyborohydride (200 mg) were added in that order to a 1 % acetic acid/chloroform solution (7 mL) of the compound (130 mg) obtained in Reference Example 2-2, and stirred for one full day at room temperature. Aqueous 1 N sodium hydroxide solution was added to the reaction liquid, and extracted with chloroform. The organic layer was dried with anhydrous sodium sulfate, and concentrated under reduced pressure. The residue was purified through silica gel column chromatography (chloroform/methanol = 90/10 to 10/1) to give the entitled compound (91 mg) as a colorless solid.
¹H-NMR (400 MHz, CDCl₃, δ ppm) : 1.55-1.67 (2H, m), 1.98-2.06 (2H, m), 2.10 (3H, s), 2.21-2.30 (2H, m), 2.96-3.03 (2H, m), 3.77 (2H, s), 3.86 (6H, s), 3.95-4.04 (1H, m), 5.95-5.98 (1H, brs), 6.87 (2H, s), 7.11-7.23 (3H, m), 7.38 (1H, d, J = 8.0 Hz), 7.74 (1H, d, J = 8.0 Hz), 8.19-8.26 (1H, brs).
ESI-MS Found : m/z 408[M+H]⁺

### Example 2-2:

### Production of 4-methyl-N-{1-[(1-methyl-1-H-indol-3-yl)methyl]-4-piperidinyl}-3,5-bis(methyloxy)benzamide

The entitled compound was obtained in the same manner as in Example 2-1, for which, however, the compound obtained in Reference Example 2-2 and 1-methyl-1H-indole-3-carbaldehyde were used, and as an additive, zinc(II) chloride and a reducing agent, sodium cyanoborohydride were used.
¹H-NMR (400 MHz, CDCl3, δ ppm) : 2.09 (3H, s), 2.02-2.20 (4H, m), 2.75-2.88 (2H, m), 3.48-3.58 (2H, m), 3.84 (3H, s), 3.86 (6H, s), 4.06-4.12 (1H, m), 4.30 (2H, s), 6.46-6.53 (1H, brs), 6.93 (2H, s), 7.26-7.33 (1H, m), 7.35-7.43 (2H, m), 7.55 (1H, d, J = 6.8 Hz).
ESI-MS Found : m/Z 422[M+H]⁺

### Example 2-3:

### Production of 4-methyl-N-(1-{[1-methyl-5-(methyloxy)-1H-indol-3-yl]methyl}-4-piperidinyl)-3,5-bis(methyloxy)benzamide

The entitled compound was obtained in the same manner as in Example 2-2, for which, however, the compound obtained in Reference Example 2-2 and 1-methyl-5-(methyloxy)-1H-indole-3-carbaldehyde were used.
¹H-NMR (400 MHz, CDCl₃,δ ppm) : 1.87-1.92 (2H, m), 2.03 (3H, s), 2.13-2.24 (2H, m), 3.12-3.28 (2H, m), 3.52-3.62 (2H, m), 3.81 (3H, s), 3.83 (6H, s), 3.85 (3H, s), 4.08-4.14 (1H, m), 4.45 (2H, s), 6.91 (1H, dd, J = 8.8, 2.0 Hz), 7.05 (2H, s), 7.21 (1H, d, J = 2.0 Hz), 7.35 (1H, d, J = 8.8 Hz), 7.40 (1H, s).
ESI-MS Found : m/Z 452[M+H]⁺

### Example 2-4:

### Production of 4-methyl-3,5-bis(methyloxy)-N-[1-({5-[(phenylmethyl)oxy]-1H-indol-3-yl}methyl)-4-piperidinyl]benzamide

The entitled compound was obtained in the same manner as in Example 2-2, for which, however, the compound obtained in Reference Example 2-2 and 5-[(phenylmethyl)oxy]-1H-indole-3-carbaldehyde were used.
¹H-NMR (400 MHz, CDCl₃, δ ppm) : 1.88-1.98 (2H, m), 2.10 (3H, s), 2.14-2.26 (2H, m), 2.39-2.50 (2H, m), 3.30-3.40 (2H, m), 3.38 (6H, s), 3.92-4.01 (1H, m), 4.42 (2H, s), 5.18 (2H, s), 6.71 (1H, dd, J = 6.8, 1.2 Hz), 6.93 (2H, s), 7.08 (1H, m), 7.18 (1H, d, J = 6.8 Hz), 7.46 (1H, s), 7.48-7.58 (5H, m), 8.51 (1H, s).
ESI-MS Found : m/Z 514[M+H]⁺

### Example 2-5:

### Production of N-{1-[(1,2-dimethyl-1H-indol-3-yl)methyl]-4-piperidinyl}-4-methyl-3,5-bis(methyloxy)benzamide

The entitled compound was obtained in the same manner as in Example 2-2, for which, however, the compound obtained in Reference Example 2-2 and 1,2-dimethyl-1H-indole-3-carbaldehyde were used.
¹H-NMR (400 MHz, MeOD, δ ppm) : 1.73-1.87 (2H, m), 2.04 (3H, s), 2.05-2.10 (2H, m), 2.48 (3H, s), 2.70-2.88 (2H, m)3.39-3.45 (2H, m), 3.72 (3H, s), 3.83 (6H, s), 3.90-4.02 (1H, m), 4.15 (2H, s), 7.04 (2H, s), 7.08 (1H, dd, J = 8.4, 7.2 Hz), 7.13 (1H, dd, J = 8.0, 7.2 Hz), 7.33 (1H, d, J = 8.4 Hz), 7.60 (1H, d, J = 8.0 Hz).
ESI-MS Found : m/z 436[M+H]⁺

### Example 2-6:

### Production of N-{1-[(1-ethyl-1H-indol-3-yl)methyl]-4-piperidinyl}-4-methyl-3,5-bis(methyloxy)benzamide

The entitled compound was obtained in the same manner as in Example 2-2, for which, however, the compound obtained in Reference Example 2-2 and 1-ethyl-1H-indole-3-carbaldehyde were used.
¹H-NMR (400 MHz, MeOD, δ ppm) : 1.46 (3H, t, J = 7.2 Hz), 1.85-1.98 (2H, m), 2.04 (3H, s), 2.14-2.25 (2H, m), 3.10-3.21 (2H, m), 3.51-3.62 (2H, m), 3.83 (6H, s), 4.04-4.14 (1H, m), 4.27 (2H, q, J = 7.2 Hz), 4.45 (2H, s), 7.04 (2H, s), 7.17 (1H, dd, J = 14.8, 7.2 Hz), 7.25 (1H, dd, J = 15.2, 7.2 Hz), 7.46 (1H, d, J = 7.6 Hz), 7.48 (1H, s), 7.72 (1H, d, J = 7.6 Hz).
ESI-MS Found : m/Z 436[M+H]⁺

### Example 2-7:

### Production of N-{1-[(1-cyclopentyl-1H-indol-3-yl)methyl]-4-piperidinyl}-4-methyl-3,5-bis(methyloxy)benzamide

The entitled compound was obtained in the same manner as in Example 2-2, for which, however, the compound obtained in Reference Example 2-2 and 1-cyclopentyl-1H-indole-3-carbaldehyde were used.
¹H-NMR (400 MHz, MeOD, δ ppm) : 1.71-1.86 (4H, m), 1.87-1.99 (4H, m), 2.00-2.04 (2H, m), 2.04 (3H, s), 2.19-2.28 (2H, m), 2.55-2.68 (2H, m), 3.19-3.25 (2H, m), 3.83 (6H, s), 3.90-4.00 (1H, m), 4.06 (2H, s), 4.56 (1H, brs), 7.04 (2H, s), 7.10 (1H, dd, J = 8.4, 7.2 Hz), 7.18 (1H, dd, J = 8.0, 7.2 Hz), 7.42 (1H, s), 7.44 (1H, d, J = 8.4 Hz), 7.66 (1H, d, J = 8.0 Hz).
ESI-MS Found : m/Z 476[M+H]⁺

### Example 2-8:

### Production of 4-methyl-3,5-bis(methyloxy)-N-(1-{[1-(2-propen-1-yl)-1H-indol-3-yl]methyl}-4-piperidinyl)benzamide formate

The entitled compound, formate was obtained in the same manner as in Example 2-2, for which, however, the compound obtained in Reference Example 2-2 and 1-(2-propen-1-yl)-1H-indole-3-carbaldehyde were used.
¹H-NMR (400 MHz, MeOD, δ ppm) : 1.82-1.98 (2H, m), 2.04 (3H, s), 2.08-2.18 (2H, m), 2.95-3.08 (2H, m), 3.42-3.53 (2H, m), 3.83 (6H, s), 4.00-4.08 (1H, m), 4.36 (2H, s), 4.80-4.90 (2H, m), 5.04 (1H, d, J = 17.2 Hz), 5.18 (1H, d, J = 10.4 Hz), 5.98-6.07 (1H, m), 7.05 (2H, s), 7.12-7.26 (2H, m), 7.41 (1H, d, J = 8.4 Hz), 7.45 (1H, s), 7.72 (1H, d, J = 8.0 Hz), 8.52 (1H, s).
ESI-MS Found : m/Z 448[M+H]⁺

### Example 2-9:

### Production of N-(1-{[1-(difluoromethyl)-1H-indol-3-yl]methyl}-4-piperidinyl)-4-methyl-3,5-bis(methyloxy)benzamide

The entitled compound, formate was obtained in the same manner as in Example 2-2, for which, however, the compound obtained in Reference Example 2-2 and 1-(difluoromethyl)-1H-indole-3-carbaldehyde were used.
¹H-NMR (400 MHz, CDCl₃ , δ ppm) : 1.88-1.96 (2H, m), 2.01 (3H, s), 2.06-2.14 (2H, m), 2.25-2.38 (2H, m), 3.49-3.58 (2H, m), 3.83 (6H, s), 4.10-4.18 (1H, m), 4.29 (2H, s), 6.77 (1H, d, J = 6.8 Hz), 6.87 (2H, s), 7.21 (1H, t, J = 60.0 Hz), 7.23-7.36 (2H, m), 7.51-7.60 (2H, m), 7.57 (1H, s).
ESI-MS Found : m/Z 458[M+H]⁺

### Example 2-10:

### Production of 4-methyl-3,5-bis(methyloxy)-N-(1-{[1-(2-propyn-1-yl)-1H-indol-3-yl]methyl}-4-piperidinyl)benzamide

The entitled compound was obtained in the same manner as in Example 2-2, for which, however, the compound obtained in Reference Example 2-2 and 1-(2-propyn-1-yl)-1H-indole-3-carbaldehyde were used.
¹H-NMR (400 MHz, MeOD, δ ppm) : 1.82-1.96 (2H, m), 2.05 (3H, s), 2.12-2.21 (2H, m), 2.90 (1H, t, J = 2.4 Hz), 3.03-3.15 (2H, m), 3.49-3.57 (2H, m), 3.84 (6H, s), 4.03-4.12 (1H, m), 4.40 (2H, s), 5.05 (2H, d, J = 2.4 Hz), 7.05 (2H, s), 7.21 (1H, t, J = 7.2 Hz), 7.28 (1H, t, J = 7.2 Hz), 7.54 (1H, d, J = 8.4 Hz), 7.57 (1H, s), 7.74 (1H, d, J = 7.6 Hz).
ESI-MS Found : m/Z 446[M+H]⁺

### Example 2-11:

### Production of 4-methyl-3,5-bis(methyloxy)-N-(1-{[1-(phenylmethyl)-1H-indol-3-yl]methyl}-4-piperidinyl)benzamide

The entitled compound was obtained in the same manner as in Example 2-2, for which, however, the compound obtained in Reference Example 2-2 and 1-(phenylmethyl)-1H-indole-3-carbaldehyde were used.
¹H-NMR (400 MHz, CDCl₃, δ ppm) : 1.97-2.06 (2H, m), 2.11 (3H, s), 2.13-2.20 (2H, m), 2.64-2.77 (2H, m), 3.40-3.49 (2H, m), 3.86 (6H, s), 4.10-4.17 (1H, m), 4.20 (2H, s), 5.32 (2H, s), 6.39 (1H, d, J = 7.5 Hz), 6.91 (2H, s), 7.10-7.18 (2H, m), 7.20-7.26 (2H, m), 7.28-7.39 (5H, m), 7.61 (1H, d, J = 6.3 Hz).
ESI-MS Found : m/Z 498[M+H]⁺

### Example 2-12:

### Production of N-[1-({1-[(6-chloro-3 -pyridinyl)methyl]-1H-indol-3-yl}methyl)-4-piperidinyl]-4-methyl-3,5-bis(methyloxy)benzamide

The entitled compound was obtained in the same manner as in Example 2-2, for which, however, the compound obtained in Reference Example 2-2 and 1-[(6-chloro-3-pyridinyl)methyl]-1H-indole-3-carbaldehyde were used.
¹H-NMR (400 MHz, MeOD, δ ppm) : 1.82-1.98 (2H, m), 2.04 (3H, s), 2.12-2.19 (2H, m), 2.98-3.10 (2H, m), 3.48-3.52 (2H, m), 3.83 (6H, s), 4.01-4.13 (1H, m), 4.39 (2H, s), 5.50 (2H, s), 7.05 (2H, s), 7.18-7.28 (2H, m), 7.36-7.42 (2H, m), 7.59 (1H, s), 7.61 (1H, d, J = 8.0 Hz), 7.76 (1H, d, J = 8.0 Hz), 8.20 (1H, s).
ESI-MS Found : m/Z 534[M+H]⁺

### Example 2-13:

### Production of N-[1-({1-[(2-chloro-1,3-thiazol-5-yl)methyl]-1H-indol-3-yl}methyl)-4-piperidinyl]-4-methyl-3,5-bis(methyloxy)benzamide

The entitled compound was obtained in the same manner as in Example 2-2, for which, however, the compound obtained in Reference Example 2-2 and 1-[(2-chloro-1,3-thiazol-5-yl)methyl]-1H-indole-3-carbaldehyde were used.
¹H-NMR (400 MHz, MeOD, δ ppm) : 1.78-1.90 (2H, m), 2.05 (3H, s), 2.08-2.17 (2H, m), 2.83-2.98 (2H, m), 3.37-3.48 (2H, m), 3.84 (6H, s), 3.99-4.08 (1H, m), 4.28 (2H, s), 5.63 (2H, s), 7.05 (2H, s), 7.18-7.38 (2H, m), 7.51 (1H, s), 7.53 (1H, d, J = 8.0 Hz), 7.56 (1H, s), 7.74 (1H, d, J = 8.0 Hz).
ESI-MS Found : m/Z 540[M+H]⁺

### Production of N-(1-{[1-ethyl-5-(methyloxy)-1H-indol-3-yl]methyl}-4-piperidinyl)-4-methyl-3,5-bis(methyloxy)benzamide

The entitled compound was obtained in the same manner as in Example 2-2, for which, however, the compound obtained in Reference Example 2-2 and 1-ethyl-5-(methyloxy)-1H-indole-3-carbaldehyde were used.
¹H-NMR (400 MHz, MeOD, δ ppm) : 1.42 (3H, t, J = 7.2 Hz), 1.72-1.84 (2H, m), 2.04 (3H, s), 2.05-2.09 (2H, m), 2.61-2.74 (2H, m), 3.30-3.35 (2H, m), 3.84 (9H, brs), 3.91-4.02 (1H, m), 4.08 (2H, s), 4.18 (2H, q, J = 7.2 Hz), 6.85 (1H, d, J = 8.4 Hz), 7.04 (2H, s), 7.18 (1H, s), 7.31 (1H, d, J = 8.4 Hz), 7.32 (1H, s).
ESI-MS Found : m/Z 466[M+H]⁺

### Example 2-15:

### Production of 4-methyl-N-(1-{[1methylethyl)-5-(methyloxy)-1H-indol-3-yl]methyl}-4-piperidinyl)-3,5-bis(methyloxy)benzamide

The entitled compound was obtained in the same manner as in Example 2-2, for which, however, the compound obtained in Reference Example 2-2 and 1-(1-methylethyl)-5-(methyloxy)-1H-indole-3-carbaldehyde were used.
¹H-NMR (400 MHz, MeOD, δ ppm) : 1.51 (6H, d, J = 6.8 Hz), 1.74-1.88 (2H, m), 2.04 (3H, s), 2.05-2.13 (2H, m), 2.68-2.82 (2H, m), 3.29-3.48 (2H, m), 3.84 (9H, brs), 3.98-4.05 (1H, m), 4.12 (2H, s), 4.65-4.73 (1H, m), 6.86 (1H, d, J = 8.0 Hz), 7.04 (2H, s), 7.18 (1H, s), 7.36 (1H, d, J = 8.0 Hz), 7.43 (1H, s).
ESI-MS Found : m/Z 480[M+H]⁺

### Example 2-16:

### Production of 4-methyl-3,5-bis(methyloxy)-N-(1-{[5-(methyloxy)-1-propyl-1H-indol-3-yl]methyl}-4-piperidinyl)benzamide

The entitled compound was obtained in the same manner as in Example 2-2, for which, however, the compound obtained in Reference Example 2-2 and 5-(methyloxy)-1-propyl-1H-indole-3-carbaldehyde were used.
¹H-NMR (400 MHz, MeOD, δ ppm) : 0.89 (3H, t, J = 7.6 Hz), 1.72-1.88 (4H, m), 2.04 (3H, s), 2.05-2.11 (2H, m), 2.61-2.74 (2H, m), 3.83 (9H, brs), 3.92-4.04 (1H, m), 4.06 (2H, s), 4.10 (2H, q, J = 7.6 Hz), 4.56 (2H, s), 6.82-6.88 (1H, m), 7.04 (2H, s), 7.29 (1H, s), 7.38-7.42 (2H, m).
ESI-MS Found : m/Z 480[M+H]⁺

### Example 2-17:

### Production of 4-methyl-N-{1-[(1-methyl-1H-indol-2-yl)methyl]-4-piperidin}-3,5-bis(methyloxy)benzamide

The entitled compound was obtained in the same manner as in Example 2-2, for which, however, the compound obtained in Reference Example 2-2 and 1-methyl-1H-indole-2-carbaldehyde were used.
¹H-NMR (400 MHz, MeOD, δ ppm) : 1.65-1.76 (2H, m), 1.95-2.01 (2H, m), 2.04 (3H, s), 2.34-2.44 (2H, m), 3.09-3.16 (2H, m), 3.80 (3H, s), 3.83 (8H, brs), 3.87-3.96 (1H, m), 6.42 (1H, s), 6.98-7.02 (1H, m), 7.05 (2H, s), 7.11-7.15 (1H, m), 7.34 (1H, d, J = 8.4 Hz), 7.47 (1H, d, J = 7.6 Hz).
ESI-MS Found : m/Z 422[M+H]⁺

### Example 2-18:

### Production of 4-methyl-N-{1-[(3-methyl-1H-indol-2-yl)methyl]-4-piperidinyl}-3,5-bis(methyloxy)benzamide

The entitled compound was obtained in the same manner as in Example 2-2, for which, however, the compound obtained in Reference Example 2-2 and 3-methyl-1H-indole-2-carbaldehyde were used.
¹H-NMR (400 MHz, MeOD, δ ppm) : 1.72-1.83 (2H, m), 2.00-2.03 (2H, m), 2.04 (3H, s), 2.31 (3H, s), 2.52-2.63 (2H, m), 3.15-3.24 (2H, m), 3.83 (6H, s), 3.90-4.02 (3H, m), 7.01 (1H, m), 7.04 (2H, s), 7.11 (1H, m), 7.32 (1H, d, J = 7.2 Hz), 7.48 (1H, d, J = 7.2 Hz).
ESI-MS Found : m/Z 422[M+H]⁺

### Example 2-19:

### Production of N-{1-[(3-chloro-1H-indol-2-yl)methyl]-4-piperidinyl}-4-methyl-3,5 bis(methyloxyl)benzamide

The entitled compound was obtained in the same manner as in Example 2-2, for which, however, the compound obtained in Reference Example 2-2 and 3-chloro-1H-indole-2-carbaldehyde were used.
¹H-NMR (400 MHz, MeOD, δ ppm) : 1.82-1.98 (2H, m), 2.05 (3H, s), 2.10-2.18 (2H, m), 2.89-2.98 (2H, m), 3.39-3.45 (2H, m), 3.84 (6H, s), 3.99-4.08 (1H, m), 4.26 (2H, s), 7.05 (2H, s), 7.14 (1H, dd, J = 8.0, 7.2 Hz), 7.25 (1H, dd, J = 8.4, 7.2 Hz), 7.41 (1H, d, J = 8.4 Hz), 7.53 (1H, d,J=8.0Hz).
ESI-MS Found : m/Z 443[M+H]⁺

### Example 2-20:

### Production of 4-methyl-3,5-bis(methyloxy)-N-(1-{[5-(methyloxy)-1H-pyrrolo[3,2-b]pyridin-2-yl]methyl}-4-piperidinyl)benzamide

The entitled compound was obtained in the same manner as in Example 2-2, for which, however, the compound obtained in Reference Example 2-2 and 5-(methyloxy)-1H-pyrrolo[3,2-b]pyridine-2-carbaldehyde were used.
¹H-NMR (400 MHz, MeOD, δ ppm) : 1.82-1.98 (2H, m), 2.04 (3H, s), 2.12-2.18 (2H, m), 2.90-2.99 (2H, m), 3.39-3.48 (2H, m), 3.83 (6H, s), 3.91 (3H, s), 4.00-4.10 (1H, m), 4.28 (2H, s), 6.62 (1H, s), 6.65 (1H, d, J = 8.8 Hz), 7.05 (2H, s), 7.72 (1H, d, J = 8.8 Hz).
ESI-MS Found : m/Z 439[M+H]⁺

### Example 2-21:

### Production of 4-methyl-3,5-bis(methyloxy)-N-{1-[(5-oxo-4,5-dihydro-1H-pyrrolo[3,2-b]pyridin-2-yl)methyl]-4-piperidinyl}benzamide

The entitled compound was obtained in the same manner as in Example 2-1, for which, however, the compound obtained in Reference Example 2-2 and 5-oxo-4,5-dihydro-1H-pyrrolo[3,2-b]pyridine-2-carbaldehyde were used, and as an additive a catalytic amount of, TFA and as a reducing agent, triethylsilane were used.
¹H-NMR (400 MHz, MeOD, δ ppm): 1.58-1.68 (2H, m), 2.01-2.08 (2H, m), 2.14 (3H, s), 3.01-3.23 (2H, m), 3.31-3.39 (2H, m), 3.63 (3H, s), 3.86 (3H, s), 3.96-4.03 (1H, m), 4.19 (2H, s), 5.92 (1H, s), 6.30 (1H, d, J = 8.0 Hz), 6.78 (2H, s), 7.76 (1H, d, J = 8.0 Hz).
ESI-MS Found : m/Z 425[M+H]⁺

### Example 2-22:

### Production of 4-methyl-N-{1-[(1-methyl-1H-indazol-3-yl)methyl]-4-piberidinyl}-3,5-bis(methyloxy)benzamide

The entitled compound was obtained in the same manner as in Example 2-2, for which, however, the compound obtained in Reference Example 2-2 and 1-methyl-1H-indazole-3-carbaldehyde were used.
¹H-NMR (400 MHz, MeOD, δppm): 1.63-1.79 (2H, m), 1.89-1.96 (2H, m), 1.98 (3H, s), 2.48-2.60 (2H, m), 3.12-3.24 (2H, m), 3.79 (6H, s), 3.81-3.88 (1H, m), 4.01 (3H, s), 4.13 (2H, s), 6.98 (2H, s), 7.13 (1H, dd, J = 8.4, 6.8 Hz), 7.37 (1H, dd, J = 8.0, 6.8 Hz), 7.48 (1H, d, J = 8.4 Hz), 7.81 (1H, d, J = 8.0 Hz).
ESI-MS Found : m/Z 423[M+H]⁺

### Example 2-23:

### Production of N-[1-(1-benzofuran-3-ylmethyl)-4-piperidinyl]-4-methyl-3,5-bis(methyloxy)benzamide

The entitled compound was obtained in the same manner as in Example 2-2, for which, however, the compound obtained in Reference Example 2-2 and 1-benzofuran-3-carbaldehyde were used.
¹H-NMR (400 MHz, CDC₃, δ ppm) : 1.92-2.05 (2H, m), 2.11 (3H, s), 2.13-2.21 (2H, m), 2.60-2.76 (2H, m), 3.30-3.42 (2H, m), 3.85 (6H, s), 4.08 (2H, s), 4.10-4.20 (1H, m), 6.43 (1H, m), 6.92 (2H, s), 7.28-7.38 (2H, m), 7.53 (1H, d, J = 7.2 Hz), 7.65 (1H, d, J = 7.2 Hz), 7.80 (1H, s). ESI-MS Found : m/Z 409[M+H]⁺

### Example 2-24:

### Production of 4-methyl-3,5-bis(methyloxy)-N-[1-(thieno[2,3-b]pyridin-2-ylmethyl)-4-piperidinyl]benzamide

The entitled compound was obtained in the same manner as in Example 2-2, for which, however, the compound obtained in Reference Example 2-2 and thieno[2,3-b]pyridine-2-carbaldehyde were used.
¹H-NMR (400 MHz, CDCl₃, δ ppm) : 1.89-2.01 (2H, m), 2.07 (3H, s), 2.10-2.16 (2H, m), 2.51-2.62 (2H, m), 3.18-3.38 (2H, m), 3.83 (6H, s), 4.06 (2H, s), 4.06-4.18 (1H, m), 6.30-6.48 (1H, m), 6.90 (2H, s), 7.29 (1H, dd, J = 8.0, 4.8 Hz), 7.35 (1H, s), 7.98 (1H, dd, J = 8.0, 1.6 Hz), 8.53 (1H, dd, J=4.8, 1.6 Hz).
ESI-MS Found : m/Z 426[M+H]⁺

### Example 2-25:

### Production of 4-methyl-3,5-bis(methyloxy)-N-{1-[(4-methyl-4H-thieno[3,2-b]pyrrol-5-yl)methyl]-4-piperidinyl]benzamide

The entitled compound was obtained in the same manner as in Example 2-2, for which, however, the compound obtained in Reference Example 2-2 and 4-methyl-4H-thieno[3,2-b]pyrrole-5-carbaldehyde were used.
¹H-NMR (400 MHz, MeOD, δ ppm) : 1.68-1.82 (2H, m), 1.98-2.03 (2H, m), 2.05 (3H, s), 2.40-2.62 (2H, m), 3.15-3.28 (2H, m), 3.79 (3H, s), 3.84 (6H, s), 3.86-4.01 (1H, m), 4.02 (2H, s), 6.40 (1H, d, J = 4.8 Hz), 6.98 (1H, d, J = 4.8 Hz), 7.05 (2H, s), 7.11 (1H, s).
ESI-MS Found : m/Z 428[M+H]⁺

### INDUSTRIAL APPLICABILITY

The compounds of the invention have an MCH-1R antagonistic effect and are useful as a preventive or remedy for metabolic disorders such as obesity, diabetes, hormone disorder, hyperlipidemia, gout, fatty liver, hepatitis, and cirrhosis; cardiovascular disorders such as stenocardia, acute/congestive heart failure, myocardial infarction, coronary atherosclerosis, renal diseases and electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence and alcoholism; reproductive disorders such as infertility, preterm labor and sexual dysfunction; other digestive disorders, respiratory disorders, cancer or pigmentation; especially as a preventive or remedy for obesity, diabetes, fatty liver, bulimia, depression or anxiety.

## Claims

1. A compound of a formula (I) or a pharmaceutically-acceptable salt thereof: 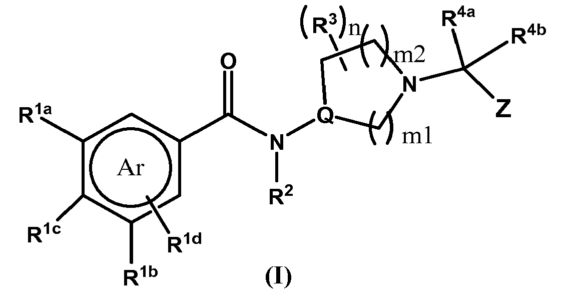 [wherein,
Q represents CH or N;
R^{1a} and R^{1b} each independently represent a halogen, a C₁₋₆ alkyloxy or a halo-C₁₋₆ alkyloxy;
R^{1c} represents a halogen, a C₁₋₆ alkyl or a halo-C₁₋₆ alkyl;
R^{1d} represents a hydrogen atom, a halogen, a C₁₋₆ alkyl, a halo-C₁₋₆ alkyl, a C₁₋₆ alkyloxy, a halo-C₁₋₆ alkyloxy, a cyano, a hydroxy-C₁₋₆ alkyl, a C₃₋₈ cycloalkyl or a hydroxy-C₃₋₈ cycloalkyl;
R² represents a hydrogen atom, a C₁₋₆ alkyl or a halo-C₁₋₆ alkyl;
R³ each independently represents a hydrogen atom, a halogen, a C₁₋₆ alkyl or a halo-C₁₋₆ alkyl; or in case where two R³'s are on different carbon atoms, they form, taken together, a substituent selected from a group of methylene, ethylene, propylene and -MCH₂-O-CH₂-, or in case where two R³'s are on the same carbon atom, they form, taken together, an oxo;
R⁴ and R^{4b} each independently represent a hydrogen atom, a C₁₋₆ alkyl or a halo-C₁₋₆ alkyl;
Z represents 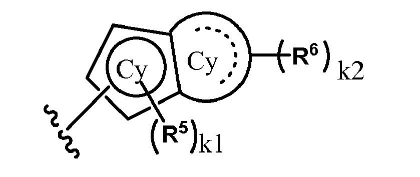 (wherein R⁵ each independently represents a hydrogen atom, a C₁₋₆ alkyl, a halo-C₁₋₆ alkyl, a C₂₋₆ alkenyl, a C₂₋₆ alkynyl, a cyano, a hydroxy-C₁₋₆ alkyl, a C₃₋₈ cycloalkyl, a hydroxy-C₃₋₈ cycloalkyl, a C₁₋₆ alkyloxycarbonyl, an aryl, a heteroaryl, an aralkyl, a heteroaralkyl or an arylsulfonyl; wherein the aryl, or the heteroaryl, or the aryl or the heteroaryl in the aralkyl, the heteroaralkyl or the arylsulfonyl may be substituted with a halogen;
R⁶ each independently represents a hydrogen atom, a halogen, a C₁₋₆ alkyl, a C₃₋₈ cycloalkyl, a halo-C₁₋₆ alkyl, a C₁₋₆ alkyloxy, a halo-C₁₋₆ alkyloxy, a C₁₋₆ alkyloxycarbonyl, a C₁₋₆ alkyloxy-C₁₋₆ alkyl, an aralkyl, an aralkyloxy, a heteroaryl, an arylthio or an arylsulfonyl; or in case where two R⁶'s are on the same hydrogen atom, they form, taken together, an oxo;
k1 indicates 1 or 2; k2 indicates 1, 2, 3 or 4; 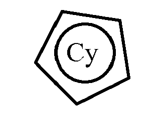 represents a 5-membered unsaturated carbon ring, or a 5-membered heteroaryl ring containing from 1 to 3, the same or different hetero atoms selected from a nitrogen atom, an oxygen atom and a sulfur atom; 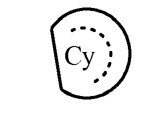 represents a 6-membered unsaturated nitrogen-containing hetero ring, a 6-membered aryl ring, or a 5 or 6-membered heteroaryl ring); 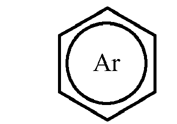 represents a benzene ring, a pyridine ring or a pyrimidine ring;
m1 and m2 each independently indicate 0, 1 or 2
n indicates an integer of from 1 to 4].

2. The compound or the pharmaceutically-acceptable salt thereof as claimed in claim 1, wherein Z is represented by a formula (Z-1), a formula (Z-2), a formula (Z-3), a formula (Z-4), a formula (Z-5) or a formula (Z-6): 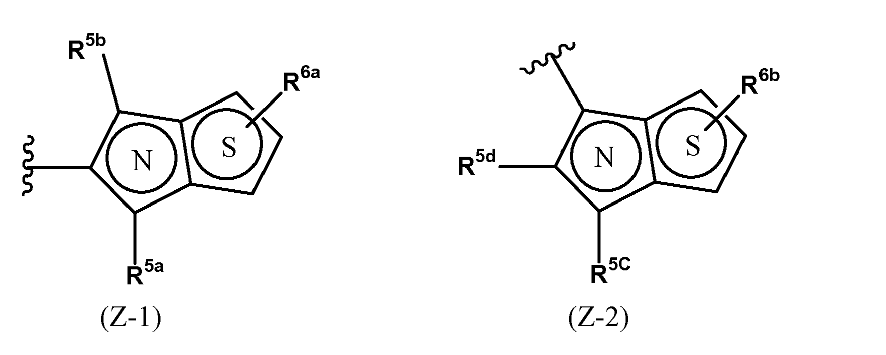 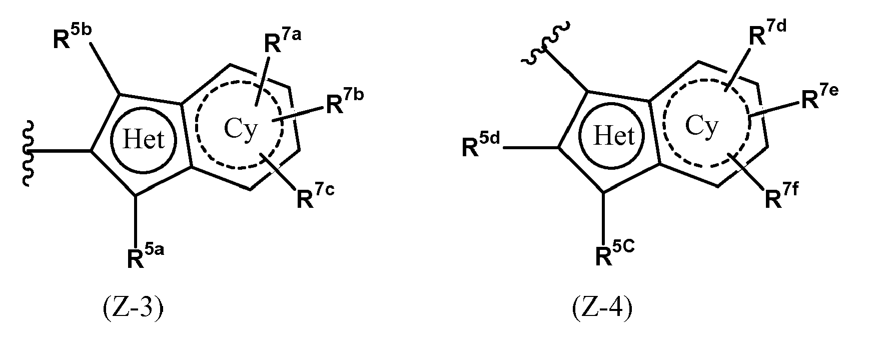 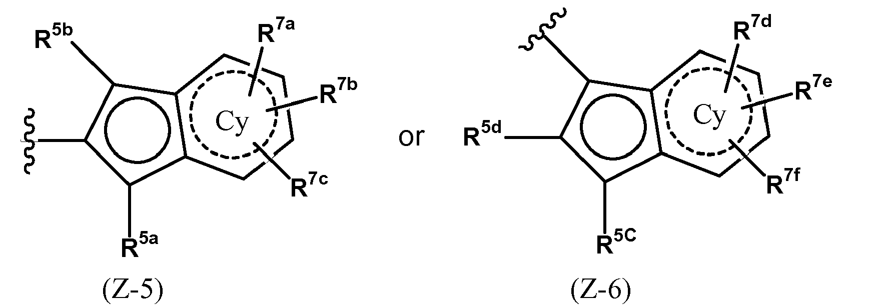
[wherein R^{5a}, R^{5b}, R^{5c} and R^{5d} each independently represent a hydrogen atom, a C₁₋₆ alkyl, a halo-C₁₋₆ alkyl, a C₂₋₆ alkenyl, a C₂₋₆ alkynyl, a cyano, a hydroxy-C₁₋₆ alkyl, a C₃₋₈ cycloalkyl, a hydroxy-C₃₋₈ cycloalkyl, a C₁₋₆ alkyloxycarbonyl, an aryl, a heteroaryl, an aralkyl, a heteroaralkyl or an arylsulfonyl, wherein the aryl, the heteroaryl, or the aryl or the heteroaryl in the aralkyl, the heteroaralkyl or the arylsulfonyl may be substituted with a halogen;
R^{6a} and R^{6b} each independently represent a hydrogen atom, a halogen, a C₁₋₆ alkyl, a C₃₋₈ cycloalkyl, a halo-C₁₋₆ alkyl, a C₁₋₆ alkyloxy, a halo-C₁₋₆ alkyloxy, a C₁₋₆ alkyloxycarbonyl, a C₁₋₆ alkyloxy-C₁₋₆ alkyl, an aralkyl, an aralkyloxy, a heteroaryl, an arylthio or an arylsulfonyl;
R^{7a}, R^{7b}, R^{7c}, R^{7d}, R^{7e} and R^{7f} each independently represent a hydrogen atom, a halogen, a C₁₋₆ alkyl, a C₃₋₈ cycloalkyl, a halo-C₁₋₆ alkyl, a C₁₋₆ alkyloxy, a halo-C₁₋₆ alkyloxy, a C₁₋₆ alkyloxycarbonyl, a C₁₋₆ alkyloxy-C₁₋₆ alkyl, an aralkyl, an aralkyloxy, a heteroaryl, an arylthio or an arylsulfonyl; or in case where R^{7a} and R^{7b}, or R^{7d} and R^{7e} are on the same carbon atom, they form, taken together, an oxo; 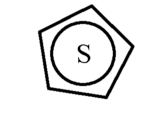 represents a 5-membered sulfur-containing heteroaryl ring optionally having a nitrogen atom in the ring; 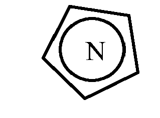 represents a 5-membered nitrogen-containing heteroaryl ring; 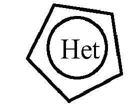 represents a 5-membered heteroaryl ring having the same or different, from 1 to 3 hetero atoms selected from a nitrogen atom, an oxygen atom and a sulfur atom; 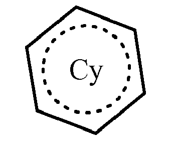 represents a 6-membered unsaturated nitrogen-containing hetero ring, a 6-membered aryl ring or a 6-membered nitrogen-containing heteroaryl ring].

3. The compound or the pharmaceutically-acceptable salt thereof as claimed in claim 1, wherein Z is represented by a formula (Z-1) or a formula (Z-2): 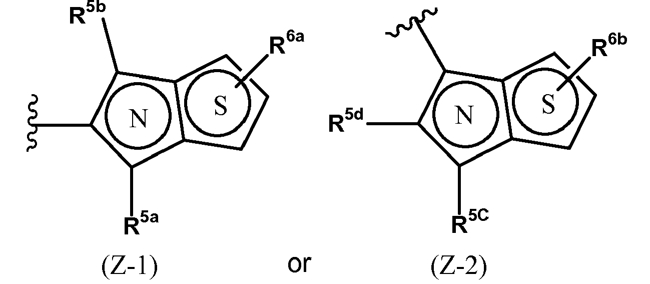 [wherein the symbols have the same meanings as in claim 2].

4. The compound or the pharmaceutically-acceptable salt thereof as claimed in claim 3, wherein the formula (Z-1) or the formula (Z-2) is selected from the following group: 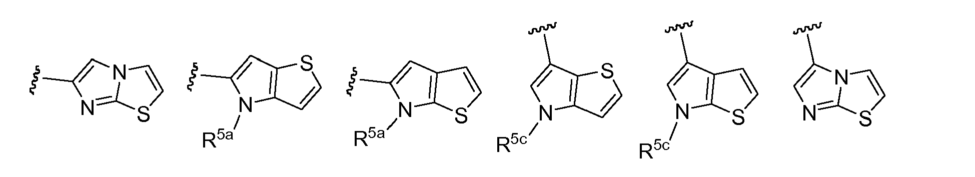 [wherein the symbols have the same meanings as in claim 2].

5. The compound or the pharmaceutically-acceptable salt thereof as claimed in claim 1, wherein Z is represented by a formula (Z-3) or a formula (Z-4): 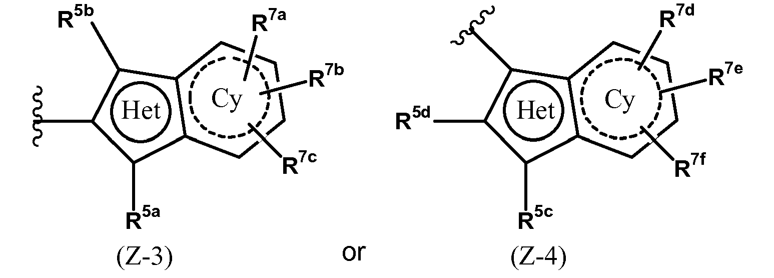 [wherein the symbols have the same meanings as in claim 2].

6. The compound or the pharmaceutically-acceptable salt thereof as claimed in claim 5, wherein the formula (Z-3) or the formula (Z-4) is selected from the following group: 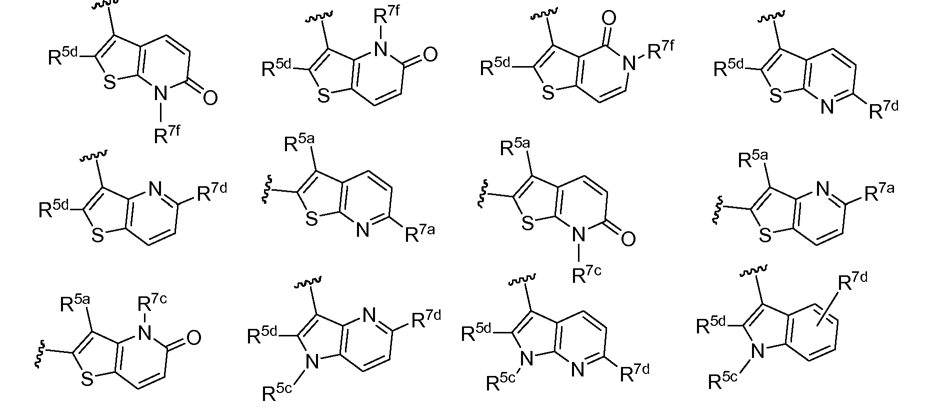 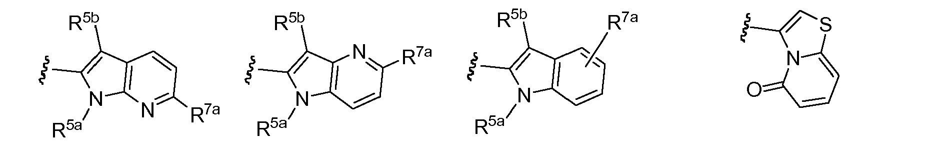 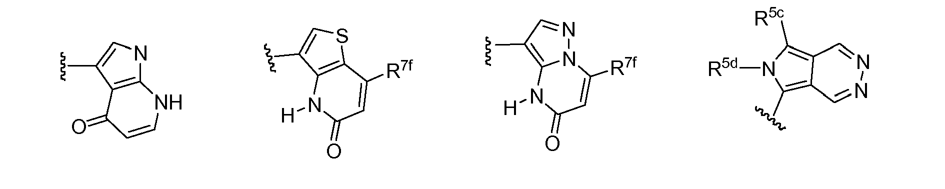 [wherein the symbols have the same meanings as in claim 2].

7. The compound or the pharmaceutically-acceptable salt thereof as claimed in claim 5, wherein the formula (Z-3) or the formula (Z-4) is the following: 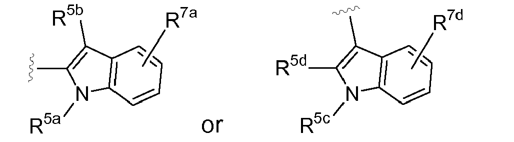 [wherein the symbols have the same meanings as in claim 2].

8. The compound or the pharmaceutically-acceptable salt thereof as claimed in claim 7, wherein R⁵ and R⁵ are a hydrogen atom or a C₁₋₆ alkyl; R^{5b} and R^{5d} are a C₁₋₆ alkyl; R^{7a} and R^{7d} are hydrogen atoms.

9. The compound or the pharmaceutically-acceptable salt thereof as claimed in claim 1, wherein Z is represented by a formula (Z-5) or a formula (Z-6): 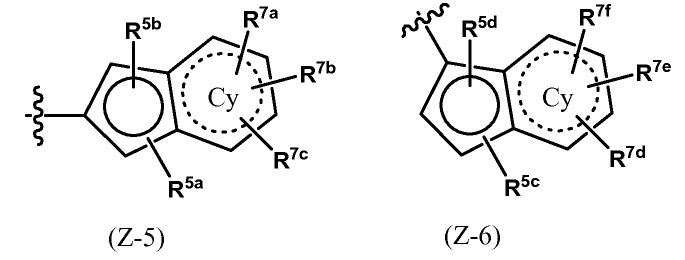 [wherein the symbols have the same meanings as in claim 2].

10. The compound or the pharmaceutically-acceptable salt thereof as claimed in any of claims 1 to 9, wherein R² is a hydrogen atom.

11. The compound or the pharmaceutically-acceptable salt thereof as claimed in any of claims 1 to 10, wherein Q is CH.

12. The compound or the pharmaceutically-acceptable salt thereof as claimed in any of claims 1 to 11, wherein Ar is a benzene ring.

13. The compound or the pharmaceutically-acceptable salt thereof as claimed in any of claims 1 to 12, wherein R^{1a} and R^{1b} are independently a halogen or a C₁₋₆ alkyloxy.

14. The compound or the pharmaceutically-acceptable salt thereof as claimed in any of claims 1 to 13, wherein R^{1c} is a halogen or a C₁₋₆ alkyl.

15. The compound or the pharmaceutically-acceptable salt thereof as claimed in any of claims 1 to 12, wherein R^{1a} and R^{1b} are independently a C₁₋₆ alkyloxy and R^{1c} is a C₁₋₆ alkyl.

16. The compound or the pharmaceutically-acceptable salt thereof as claimed in any of claims 1 to 15, wherein m1 is 2, and m2 is 2.

17. The compound or the pharmaceutically-acceptable salt thereof as claimed in any of claims 1 to 16, wherein R³ is a hydrogen atom, and n is 1.

18. A melanin concentrating hormone receptor antagonist comprising a compound or a pharmaceutically-acceptable salt thereof of any of claims 1 to 17 as the active ingredient.

19. A pharmaceutical composition containing a medicinally-acceptable additive and a compound or a pharmaceutically-acceptable salt thereof of any of claims 1 to 17.

20. A preventive or remedy comprising a compound or a pharmaceutically-acceptable salt thereof of any of claims 1 to 17 as the active ingredient, for obesity, diabetes, fatty liver, bulimia, depression or anxiety.
